# EUROPEAN PATENT APPLICATION

(11) **EP 2 151 434 A1**
(43) Date of publication of application: **10.02.2010**
(21) Application number: 08305450.2
(22) Date of filing: 05.08.2008
(51) Int. Cl.: C07D 231/20, A61K 31/415, A61P 3/10, A61P 29/00

(54) **Alkoxypyrazoles and the process for their preparation**

(71) Applicant: Institut Pasteur, 75015 Paris (FR); Centre National de la Recherche Scientifique (CNRS), 75016 Paris (FR)
(72) Inventor: Janin, Yves, 75005, PARIS (FR); Guillou, Sandrine, 94700, MAISON ALFORT (FR)
(74) Representative: Warcoin, Jacques

(57) **Abstract**

The present invention relates to a process for the preparation of alkoxypyrazoles and new alkoxypyrazole compounds.

## Description

The present invention relates to synthesis of 3-alkoxypyrazoles which can lead to many derivatives including pyrazol-3-ones.

In the last 20 years, the Suzuki-Miyaura^{1, 2} aryl-aryl cross coupling reaction has probably increased by at least an order of magnitude the number of compounds that have been synthesised and tested in many medicinal chemistry research programs. Indeed, many successful research programs in the pharmaceutical industry owe their origin to the elaboration of original core scaffolds "dressed" with substituents fine-tuned to bind known enzyme or receptor pockets or to mimic series of compounds with a known biological activity.

In the same way, heterocyclic compounds particularly five membered heterocycles have attracted the attention of pharmaceutical community over the years due to their therapeutic value.

In recent years, there has been a growing interest in discovering new pyrazoles having advantageous biological properties.

Found among these compounds, in particular, are pyrazol-3-ones having applications in the field of the treatment or prevention of diseases such as diabetes, inflammatory diseases, prion diseases, and of the treatment of pain.

Nineteen pyrazolone derivatives have been shown to inhibit PrP-res (resistant prion protein) accumulation. The most effective compound was 2-phenyl-5-(4-nitrophenyl)-pyrazol-3-one³.

1,2-dihydro-4-[[4-(methylthio)phenyl]methyl]-5-(trifluoromethyl)-3H-pyrazol-3-one has been shown to be a potent antihyperglycemic agent⁴.

Propyphenazone (1,2-dihydro-1,5-dimethyl-4-(1-methylethyl)-2-phenyl-3H-pyrazol-3-one) is a nonsteroidal anti-inflammatory drug frequently used as mild analgesic medicament⁵.

5-alkyl-4-(arylmethyl)pyrazol-3-ones have been shown to be the most promising new class of potential antidiabetic agent⁶.

The new immunosuppressant 2-(4'-chlorophenyl)benzo-thiopyrano-(4,3-c)pyrazol-3-one has been characterized⁷.

Moreover, few pyrazole-featuring compounds⁸⁻¹⁰ have been reported bearing an antimicrobial activity.

The pyrazole ring system cannot be considered as an original scaffold. For instance, the analgesic N1-phenyl-pyrazol-5-one antipyrine was first prepared in 1892. However, the core pyrazol-3-one (5) structure with at least three potential nucleophilic centres (the two nitrogens and the oxygen) and two carbons is still an attractive heterocycle. Many of these reactive centres may lend themselves to chemical transformations with a potential for "quick" chemical diversity attainable in few reaction steps. This number of reactive centres, although desirable for chemical diversity, does force the chemist to design chemical transformations involving protection and deprotection steps to insure their regioselectivity. In the case of the pyrazol-3-ones this is a problem as the protection steps themselves also face this issue of regioselectivity. From the many N-aryl or N-alkyl hydrazines, the age-old Knorr reaction³¹⁻³³ provided an easy access to many N1-subtituted pyrazol-5-ones. On the other hand, the isomeric N1-subtituted pyrazol-3-ones are less easy to prepare. This aspect is actually illustrated by a 2007 chemical data base survey. Out of the 156278 compounds featuring a pyrazolone moiety ever reported, some 113469 are N1-aryl-pyrazol-5-ones and "only" 9997 are the isomeric N1-aryl-pyrazol-3-ones. This last number actually includes compounds with a substituent on the other nitrogen (4189) as well as ring-fused 1-arylindazol-3-ones (138). In any case, from a selective O-alkylation of compound (**5**) to give the O-protected chemical intermediates (**6**) one could imagine selective chemistry on one of the pyrazole nitrogens. However, only two papers describe the selective O-alkylation of (**5**) or further chemical transformations.^{34, 35} It is only quite recently that the problem of selective oxygen protection of pyrazolones such as (**5**) was breached.

Thus, there exist important needs for a new process for a simple access to 3-alkoxypyrazoles and then to N1-substituted pyrazol-3-ones.

The patent FR 2862647 describes the generalization of processes^{8, 9} involving a transient N-carboxylation⁸ or N-acetylation⁹ prior to a then selective O-alkylation.

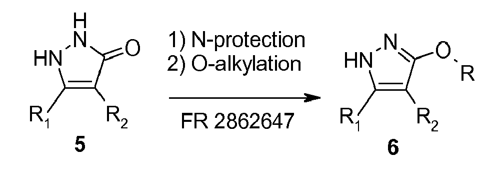

The present inventors have shown an alternative which shunts the three following steps : preparation of the pyrazol-3-one, its transient N-protection followed by its selective O-alkylation. They prepared the readily O-protected ethyl 3-ethoxy-1H-pyrazole-4-carboxylate (**8**) in one step from diethyl 2-(ethoxymethylene)malonate (**7**).

From O-protected ethyl 3-ethoxy-1H-pyrazole-4-carboxylate (**8**), the present inventors also suited few chemical transformations. These chemical transformations insured the design of accesses to fully original chemical entities.

First, from the O-protected ethyl 3-ethoxy-1H-pyrazole-4-carboxylate (**8**) and its 5-haloderivatives, they prepared N1-aryl 3-alkoxypyrazoles.

Second, the inventors investigated C4- and C5-carbon-carbon forming reactions.

Therefore, the first object of the present invention relates to a process for the preparation of alkoxypyrazoles of formula (I) or (I') 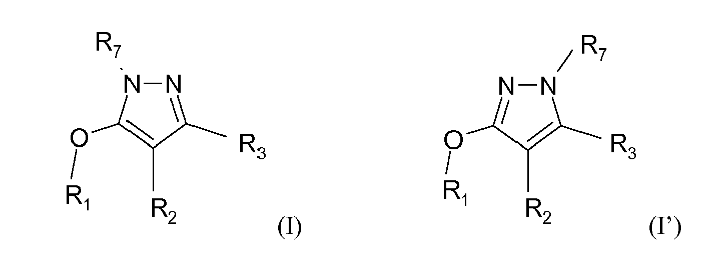 in which
R₁ stands for a radical selected from the group consisting of C₁-C₆ alkyl, C₂-C₆ alkenyl, C₂-C₆ alkynyl and (C₆-C₁₀) aryl (C₁-C₆) alkyl;
R₂ stands for a radical selected from the group consisting of hydrogen, halogen, -C(O)-O-(C₁-C₆) alkyl, -C(O)-O(C₂-C₆) alkenyl, -C(O)-O-(C₂-C₆)alkynyl;
R₃ stands for a radical selected from the group consisting of hydrogen, halogen, C₁-C₆ alkyl, C₂-C₆ alkenyl, C₂-C₆ alkynyl and (C₆-C₁₀)aryl(C₁-C₆) alkyl, each optionally substituted by R₅;
R₅ is a radical selected from the group consisting of halogen, oxygen, hydroxyl, C₁-C₆ alkyl and C₁-C₆ alkoxy;
R₇ stands for a radical selected from the group consisting of H, C₁-C₆ alkyl, C₆-C₁₀ aryl, (C₆-C₁₀)aryl(C₁-C₆) alkyl, C₆-C₁₀ heteroaryl or (C₆-C₁₀)heteroaryl(C₁-C₆)alkyl, each optionally substituted by a halogen;

comprising the successive following steps:
(a) reacting a derivative of formula (II) 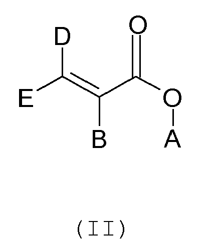
   in which A stands for a radical selected from the group consisting of C₁-C₆ alkyl, C₂-C₆ alkenyl, C₂-C₆ alkynyl and (C₆-C₁₀)aryl(C₁-C₆) alkyl;
   B stands for a radical selected from the group consisting of hydrogen, -C(O)-O-(C₁-C₆)alkyl, -C(O)-O-(C₂-C₆)alkenyl, -C(O)-O-(C₂-C₆) alkynyl;
   D stands for a radical selected from the group consisting of hydrogen, C₁-C₆ alkyl, C₂-C₆ alkenyl, C₂-C₆ alkynyl and (C₆-C₁₀)aryl(C₁-C₆) alkyl;
   E stands for a radical O-F or NR_{α}R_{β},
   F stand for a radical selected from the group consisting of H, C₁-C₆ alkyl C₂-C₆ alkenyl, C₂-C₆ alkynyl and (C₆-C₁₀) aryl (C₁-C₆) alkyl;
   R_{α} and R_{β}, identical or different, stand for a radical selected from the group consisting of H, C₁-C₆ alkyl or C₆-C₁₀ aryl or R_{α} and R_{β} form together an C₄-C₆ alkylene radical, an C₄-C₆ alkenylene, an C₄-C₆ heteroalkylene radical or an C₄-C₆ heteroalkenylene radical; with 1 to 1.5 molar equivalents of R₇-NH-NH₂, xHX₂, in which R₇ is as defined for compound of formula (I) or (I'), X₂ is Cl or Br and x is 1 or 2;
   in an alkylalcohol R₈OH as a solvent, in which R₈ is a radical selected from the group consisting of C₁-C₆ alkyl, C₂-C₆ alkenyl, C₂-C₆ alkynyl and (C₆-C₁₀)aryl(C₁-C₆)alkyl, (C₆-C₁₀)heteroaryl(C₁-C₆)alkyl;
   to obtain compounds of formula (III) or (III') 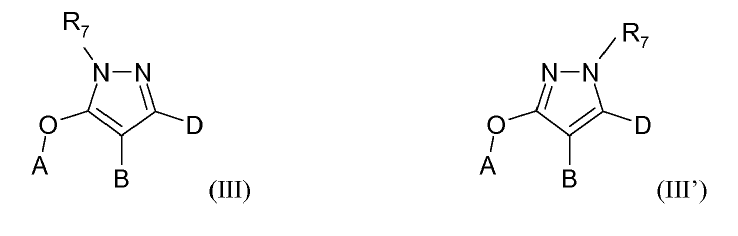
(b) optionally reacting said alkoxypyrazole of formula (III') wherein R₇ = H obtained in step (a) with X to prepare compounds of formula (IV) or (IV') 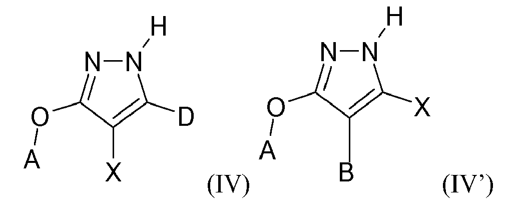 in which
   X represents Br, Cl, I or F;
   A, B and D are as defined above;
(c) recovering the alkoxypyrazole of formula (I) or (I').

Advantageously, the molar ratio of said compound of formula (II) to said R₇-NH-NH₂, xHX₂, x is 1 or 2, X₂ is Cl or Br, is close to 1, preferably comprised between 0.95 and 1.1, more preferably between 1 and 1.05.

In a preferred embodiment, said compound of formula (II) is reacted with 1.05 molar equivalents of R₇-NH-NH₂, xHCl, in which R₇ and x are as defined above.

In a preferred embodiment, E is NR_{α}R_{β} in the compound of formula (II) and the reaction in step (a) is run with R₇-NH-NH₂, 2HCl.

In another preferred embodiment, E is O-F and F is C₁-C₆ alkyl in the compound of formula (II) and the reaction in step (a) is run with R₇-NH-NH₂, HCl.

Advantageously, R₇ is hydrogen or a phenyl, benzyl or methyl group.

Preferably, said R₇-NH-NH₂, xHCl is R₇-NH-NH₂, HCl (x is 1), more preferably is said R₇-NH-NH₂, xHCl is NH₂-NH₂, HCl.

In one embodiment, the reaction in step (a) is run at the boiling point of the solvent, preferably for 16 hours.

In another embodiment, the reaction in step (a) is run overnight at room temperature.

Preferably, the reaction in step (a) is run in ethanol (R₈ is an ethyl group) or isopropanol, more preferably ethanol.

In a preferred embodiment, 1.05 equivalents of hydrazine monohydrochloride are used in boiling ethanol.

In a preferred embodiment, said compound of formula (II) is diethyl 2-(ethoxymethylene)malonate.

In a preferred embodiment, A stands for a C₁-C₆ alkyl radical, preferably stands for methyl or ethyl.

In a preferred embodiment, B stands for hydrogen, a - COOH or -COOEt radical.

In a preferred embodiment, D stands for a radical selected from the group consisting of hydrogen or C₁-C₆ alkyl, preferably methyl.

In a preferred embodiment, E stands for a radical selected from the group consisting of NMeMe or O-ethyl.

Preferably, R₇ is a hydrogen atom.

Advantageously, in step (b) the compounds of formula (IV) or (IV') are prepared using N-X-succinimide in boiling cyclohexane.

Another object of the present invention relates to alkoxypyrazoles of formula I or I' obtainable by the process according to the invention, except compounds of formula (I') wherein simultaneously
R₁ is ethyl, R₂ is H, R₃ is phenyl, R₇ is H;
R₁ is ethyl, R₂ is -COOH,-COOEt or H, R₃ and R₇ are H;
R₁ is methyl, R₂, R₃ and R₇ are H;
R₁ is ethyl, R₂ is-COOEt, R₃ is H, R₇ is methyl
and compounds of formula (I) wherein simultaneously
R₁ is ethyl, R₂ is -COOEt, R₃ is H and R₇ is phenyl;
R₁ is ethyl, R₂ is -COOEt, R₃ is H and R₇ is methyl.

Another object of the present invention relates to alkoxyppyrazoles of formula (V) or (V') 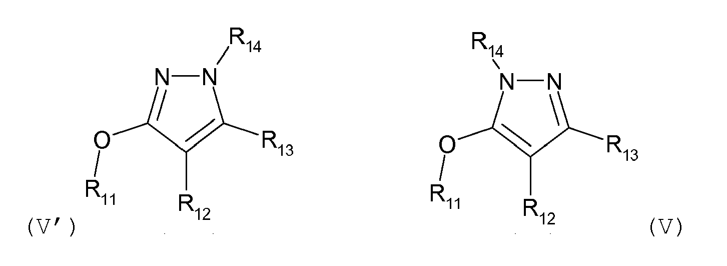 in which
R₁₁ stands for a radical selected from the group consisting of methyl or ethyl,
R₁₂ stands for a radical selected from the group consisting of a hydrogen, halogen, benzyl, a phenyl optionally substituted by Cl or Br, -COOH,-COOEt;
R₁₃ stands for a radical selected from the group consisting of hydrogen, halogen, methyl, phenyl, benzyl, morpholino, R₁₄ stands for a radical selected from the group consisting of hydrogen, phenyl, benzyl, mesyl, -CH₃SO₂ or methyl, except compounds of formula (V') wherein simultaneously R₁₁ is ethyl, R₁₂ is H, R₁₃ is phenyl, R₁₄ is H;
R₁₁ is ethyl, R₁₂ is -COOH,-COOEt or H, R₁₃ and R₁₄ are H; R₁₁ is methyl, R₁₂, R₁₃ and R₁₄ are H;
R₁₁ is ethyl, R₁₂ is-COOEt, R₁₃ is H, R₁₄ is methyl
and compounds of formula (V) wherein simultaneously
R₁₁ is ethyl, R₁₂ is -COOEt, R₁₃ is H and R₁₄ is phenyl;
R₁₁ is ethyl, R₁₂ is -COOEt, R₁₃ is H and R₁₄ is methyl.

In a preferred embodiment, alkoxypyrazoles of formula (V) or (V') are the following ones, in which
R₁₁ stands for a radical selected from the group consisting of methyl or ethyl,
R₁₂ stands for a radical selected from the group consisting of a hydrogen, halogen, -COOH,-COOEt;
R₁₃ stands for a radical selected from the group consisting of hydrogen, halogen, methyl,
R₁₄ stands for a radical selected from the group consisting of hydrogen, phenyl, benzyl or methyl,
except compounds of formula (V') wherein simultaneously
R₁₁ is ethyl, R₁₂ is H, R₁₃ is phenyl, R₁₄ is H;
R₁₁ is ethyl, R₁₂ is -COOH,-COOEt or H, R₁₃ and R₁₄ are H;
R₁₁ is methyl, R₁₂, R₁₃ and R₁₄ are H;
R₁₁ is ethyl, R₁₂ is-COOEt, R₁₃ is H, R₁₄ is methyl
and compounds of formula (V) wherein simultaneously
R₁₁ is ethyl, R₁₂ is -COOEt, R₁₃ is H and R₁₄ is phenyl;
R₁₁ is ethyl, R₁₂ is -COOEt, R₁₃ is H and R₁₄ is methyl.

The preferred alkoxypyrazoles according to the invention of formula (V') are the following ones, in which
R₁₁ is ethyl, R₁₃ is phenyl, R₁₄ is H, R₁₂ is -COOH, -COOEt, 4-BrC₆H₄ or 4-ClC₆H₄;
R₁₁ is ethyl, R₁₃ and R₁₄ are H, R₁₂ is phenyl, I, 4-ClC₆H₄, 2-ClC₆H₄, -CH₂CH₂C₆H₅, 4-BrC₆H₄ or -CH₂C₆H₅;
R₁₁ is ethyl, R₁₂ is COOEt, R₁₄ is H, R₁₃ is Br, I, methyl;
R₁₁ is ethyl, R₁₂ is -COOEt, R₁₄ is phenyl and R₁₃ is H, I, Br or phenyl;
R₁₁ is ethyl, R₁₂ is I, R₁₃ is H, R₁₄ is phenyl;
R₁₁ is ethyl; R₁₃ is H, R₁₄ is phenyl, R₁₂ is H or phenyl;
R₁₁ is ethyl, R₁₃ is Br, R₁₄ is H, R₁₂ is 4-BrC₆H₄ or 4 - ClC₆H₄;
R₁₁ is ethyl, R₁₂ is -COOH, R₁₃ and R₁₄ are phenyl;
R₁₁ is ethyl, R₁₂ is -COOEt, R₁₃ is H, R₁₄ is -CH₂C₆H₅;
R₁₁ is ethyl, R₁₂ is I, R₁₃ is H, R₁₄ is -CH₃SO₂;
R₁₁ is ethyl, R₁₂ is -COOEt, R₁₃ is morpholino, R₁₄ is H;
R₁₁ is ethyl, R₁₂ is -COOEt, R₁₃ is I, R₁₄ is -CH₃SO₂;
R₁₁ is ethyl, R₁₂ is H, R₁₃ is I, R₁₄ is H;
R₁₁ is ethyl, R₁₂ is H, R₁₃ is benzyl, R₁₄ is H;
R₁₁ is ethyl, R₁₂ is -COOH, R₁₃ is H, R₁₄ is phenyl;
R₁₁ is ethyl, R₁₂ is H, R₁₃ is phenyl, R₁₄ is phenyl;
R₁₁ is ethyl, R₁₂ is I, R₁₃ is H, R₁₄ is mesyl;
R₁₁ is ethyl, R₁₂ is -COOEt, R₁₃ is I, R₁₄ is mesyl.

The preferred alkoxypyrazoles according to the invention of formula (V) are the following ones, in which
R₁₁ is ethyl; R₁₃ is H, R₁₄ is phenyl, R₁₂ is H or I;
R₁₁ is ethyl; R₁₂ is -COOEt, R₁₃ is I or Br, R₁₄ is phenyl;
R₁₁ is ethyl; R₁₂ is -COOEt, R₁₃ is H, R₁₄ is -CH₂C₆H₅;
R₁₁ is ethyl; R₁₂ is I, R₁₃ is H, R₁₄ is mesyl;
R₁₁ is ethyl; R₁₂ is -COOEt, R₁₃ is I, R₁₄ is mesyl;
R₁₁ is ethyl, R₁₂ is -COOEt, R₁₃ is I, R₁₄ is -CH₃SO₂;
R₁₁ is ethyl, R₁₂ is I, R₁₃ is H, R₁₄ is -CH₃SO₂.

### Definitions

The abbreviations encountered are defined as follows: H is hydrogen,
I is iodide, Br is bromide, Cl is chloride, F is fluoride Et is ethyl.

Within the meaning of this invention, "halogen" is understood to mean an atom of fluorine, bromine, chlorine or iodine.

Within the meaning of this invention, "C₁-C₆ alkyl" group is understood to mean a saturated, linear or branched hydrocarbon chain comprising from 1 to 6 carbon atoms, in particular the methyl, ethyl, n-propyl, isopropyl, n-butyl, iso-butyl, sec-butyl, tert-butyl, n-pentyl, n-hexyl groups.

Within the meaning of this invention, "C₂-C₆ alkenyl" group is understood to mean a linear or branched hydrocarbon chain comprising at least one double bond and comprising from 2 to 6 carbon atoms, e.g., such as an ethenyl (vinyl) or propenyl group.

Within the meaning of the invention, "C₂-C₆ alkynyl" group is understood to mean a linear or branched hydrocarbon chain comprising at least one triple bond and comprising from 2 to 6 carbon atoms, e.g., such as an ethynyl or propynyl group.

Within the meaning of this invention, "aryl" group is understood to mean an aromatic group preferably comprising from 5 to 10 carbon atoms and including one or more fused rings, e.g., such as a phenyl or naphtyl group. This is advantageously phenyl.

Within the meaning of the invention, "heteroaryl" group is understood to mean any aryl group as defined above wherein one or more carbon atoms have been replaced by one or more heteroatoms, advantageously 1 to 4, and even more advantageously 1 to 2, e.g., such as sulphur, nitrogen or oxygen atoms. Examples of heteroaryl groups are the furyl, thiophenyl, pyrrolyl, pyridyl, pyrimidyl, pyrazolyl, imidazolyl, tetrazolyl or else indyl groups.

Within the meaning of this invention, "aryl-(C₁-C₆)-alkyl" group is understood to mean any aryl group as defined above, which is bound to the molecule by means of a (C₁-C₆)-alkyl group as defined above. In particular, a group such as this can be a benzyl group.

Within the meaning of this invention, "C₄-C₆ alkylene" group is understood to mean a saturated, linear or branched hydrocarbon chain comprising from 4 to 6 carbon atoms which forms a saturated hydrocarbon ring having 5 to 7 members, with the nitrogen atom to which it is linked.

Within the meaning of this invention, "C₄-C₆ alkenylene" group is understood to mean a linear or branched hydrocarbon chain comprising at least one double bond and comprising from 4 to 6 carbon atoms which forms a saturated hydrocarbon ring having 5 to 7 members, with the nitrogen atom to which it is linked.

Within the meaning of this invention, "C₄-C₆ heteroalkylene" group is understood to mean any alkylene group as defined above wherein one or more carbon atoms have been replaced by one or more heteroatoms, advantageously 1 to 2, and even more advantageously 1, e.g., such as sulphur, nitrogen or oxygen atoms, advantageously nitrogen.

Within the meaning of this invention, "C₄-C₆ heteroalkenylene" group is understood to mean any alkenylene group as defined above wherein one or more carbon atoms have been replaced by one or more heteroatoms, advantageously 1 to 2, and even more advantageously 1, e.g., such as sulphur, nitrogen or oxygen atoms, advantageously nitrogen.

The invention will be better understood upon reading the following examples, these examples serving solely to illustrate the invention.

### Examples

### Example 1 : Preparation of 3-alkoxypyrazoles

### The features of the devices used to conduct analyses of all of the compounds described in Example 1 are indicated hereinbelow:

A Biotage initiator 2 microwave oven was used for of the reactions requiring microwaves irradiations. The ¹H NMR and ¹³C NMR spectra were recorded on a Bruker Avance 400 spectrometers at 400 MHz and 100 MHz, respectively. Unless otherwise noted, CDCl₃ was the solvent used. Shifts (δ) are given in ppm with respect to the TMS signal and coupling constants (J) are given in Hertz. Column chromatography were performed either with Merck silica gel 60 (0.035 - 0.070 mm) or neutral alumina containing a suitable proportion of water, using a solvent pump operating at pressure between 2 and 7 bar (25-50 mL/mn) and an automated collecting system driven by a UV detector set to 254 nm unless stated otherwise (i.e. if ethylacetate was used then it would be set to 280 nm). Sample deposition was always carried out by absorption of the mixture to be purified on a small amount of the solid phase followed by its deposition of the top of the column. The low resolution mass spectra were obtained on an Agilent 1100 serie LC/MSD system using an atmospheric electrospray ionisation system and the high resolution mass spectroscopy spectra (HRMS) were obtained using a Waters Micromass Q-Tof with an electrospray ion source.

### Preparation of compounds 8 and 9

Diethylethoxymethylenemalonate (**7**) (37.4 g, 0.172 mol) and hydrazine hydrochloride (12.2 g, 0.178 mol) were refluxed in ethanol (500 mL) for 16 hours. The solvent was removed under reduced pressure, the residue was dispersed in water (500 ml) and slowly made basic by the addition of solid sodium hydrogencarbonate. The aqueous phase was extracted with dichloromethane; this organic phase was washed with a 1N solution of sodium hydrogencarbonate three times, dried over sodium sulfate and concentrated to dryness to yield compound **8** as an oil that solidified very slowly (13.22 g, 41 %). The aqueous phase was cautiously made acid with concentrated hydrochloric acid, saturated with sodium chloride and the resulting precipitate was filtered, washed with water and dried under vacuum while heating at 60 °C to yield compound **9** (10 g, 37 %) as a white powder.

Alternative preparations of compound **8 _{:}** diethyl 2-((dimethylamino)methylene)malonate (**6**)¹³ (0.25 g, 1.16 mmol) and hydrazine dihydrochloride (0.12 g, 1.16 mmol) were refluxed in ethanol (30 mL) for five hours. This was concnetrated to dryness and dispersed in dichloromethane. This was washed with a 1 N sodium hydrogenocarbonate, dried over sodium sulfate and concentrated to dryness to yield compound **8** (0.09 g; 42 %) as described below.

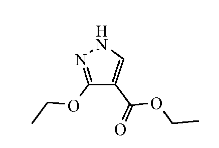

Ethyl 3-ethoxy-1H-pyrazole-4-carboxylate (**8**): ¹H (CDCl₃): 1.35 (t, 3H, J = 7.1); 1.46 6 (t, 3H, J = 7.1); 4.30 (q, 2H, J = 7.1); 4.36 (q, 2H, J = 7.1); 7.89 (s, 1H) ; 11.4 (s(1), 1H). ¹³C (CDCl₃): 14.3; 14.5; 60.1; 65.3; 99.0; 134.0; 162.3; 163.8. HRMS: Calcd. for C₈H₁₂N₂O₃ + H : 185.0926. Found: m/z, 185.0971.

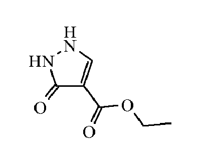

Ethyl 3-oxo-2,3-dihydro-1H-pyrazole-4-carboxylate (**9**)^{11, 8}: ¹H (DMSO-*d₆*): 1.23 (t, 3H, J = 7.1); 4.15 (q, 2H, J = 7.1); 7.90 (s(1), 1H); 10.20 (s(1), 1H). ¹³C (DMSO-*d₆;* D1 set to 10s): 19.6; 64.1; 102.2; 139.5 (1); 165.2 (1); 168.0. 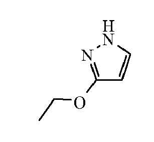 3-Ethoxy-1H-pyrazole (**10**): Diethylethoxymethylenemalonate (38.9 g, 0.179 mol) and hydrazine hydrochloride (12.69 g, 0.185 mol) were refluxed in ethanol (500 mL) for 16 hours. The solvent was removed under reduced pressure, the residue was dispersed in 6N hydrochloric acid (100 ml) and heated to reflux until the end of the carbon dioxide evolution (4 hours in the present case). The aqueous phase and slowly made basic by the addition of sodium hydrogenocarbonate and extracted with ethyl acetate four times. The organic phase was washed with a 1 N solution of sodium hydrogencarbonate once, with brine once, dried over sodium sulfate and concentrated to dryness to yield compound **10** as an oil (7.77 g, 38 %). ¹H (CDCl₃): 1.43 (t, 3H, J = 7.0); 4.23 (q, 2H, J = 7.0); 5.74 (d, 1H, J = 2.5); 7.37 (d, 1H, J = 2.5); 9.40 (s(l), 1H). ¹³C (CDCl₃): 14.9; 64.9; 90.3; 130.2; 167.7. HRMS: Calcd. for C₅H₈N₂O + H : 113.0715. Found: m/z, 113.0788.

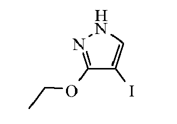

3-Ethoxy-4-iodo-1H-pyrazole (**11**): Compound **10** (8.37 g, 0.074 mol), sodium iodide (11.2 g, 0.082 mol) and potassium carbonate (40 g, 0.289 mol) were dissolved in water (400 mL) and ethanol (100 mL). To this was added iodine (28.5 g, 0.112 mol). The resulting suspension was stirred for 90 minutes hour, decolorized (if necessary) with sodium bisulfite and diluted with brine (400 mL). This was filtered, washed with water and dried under vacuum while heating at 70 °C in a large Petri dish in order to sublimate the iodoform also occurring in this reaction to yield compound **11** as an off-white solid (16.2 g; 91 %). ¹H (CDCl₃): 1.44 (t, 3H, J = 7.1); 4.31 (q, 2H, J = 7.1); 7.42 (s, 1H); 9.35 (s(l), 1H). ¹³C (CDCl₃): 14.8; 44.2; 65.5; 134.3; 163.2. HRMS: Calcd. for C₅H₇N₂OI + H : 238.9681. Found: m/z, 238.9722.

Preparation of compound **12a** or **12b:** Compound **8** (0.74 g; 4.02 mmol) and N-bromosuccinimide (0.75 g; 4.22 mmol) or N-iodosuccinimide (0.94 g, 4.22 mmol) were refluxed in cyclohexane (50 mL) for 30 minutes or 24 hours. The resulting suspension was concentrated to dryness and purified by a chromatography over silica gel (dichloromethane / ethanol 98-2) to yield compound **12a** (0.75 g; 60 %) or **12b** (0.5 g; 47 %) .

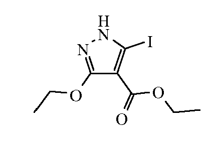

Ethyl 3-iodo-5-ethoxy-1H-pyrazole-4-carboxylate (**12a**): Obtained as an oil that slowly solidified. ¹H (CDCl₃): 1.36 (t, 3H, J = 7.0); 1.39 (t, 3H, J = 7.0); 4.31 (m, 4H). ¹³C (CDCl₃; two broad signals only visible if D1 is increased to 10): 14.2; 14.6; 60.4; 65.5; 87.0; 103.3; 161.9; 162.0. HRMS: Calcd. for C₈H₁₁IN₂O₃ + H : 332.9712. Found: m/z, 332.9722.

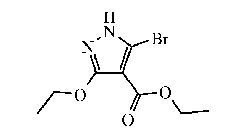

Ethyl 3-bromo-5-ethoxy-1H-pyrazole-4-carboxylate (**12b**): Obtained as white crystals. ¹H (CDCl₃): 1.40 (t, 3H, J = 7.0); 1.48 (t, 3H, J = 7.0); 4.33 (m, 4H); 9.5 (s(l), 1H). ¹³C (CDCl₃; two broad signals only visible if D1 is increased to 10): 14.2; 14.5; 60.4; 65.7; 99.0; 119.5 (br); 161.6 (br); 162.0. HRMS: Calcd. for C₈H₁₁BrN₂O₃ + H : 263.0031. Found: m/z, 263.0049.

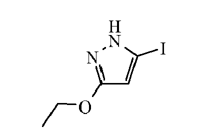

Ethyl 3-iodo-5-ethoxy-1H-pyrazole-4-carboxylate (**13**): Compound **12a** (0.99 g, 3.2 mmol) was dispersed in 6N hydrochloric acid (20 mL) and heated to reflux for 4 hours. The aqueous phase was diluted in water, slowly made basic by the addition of concentrated ammonia and extracted with ethyl acetate four times. The organic phase was washed with brine once, with brine once, dried over sodium sulfate and concentrated to dryness to yield compound **13** as an oil (0.52 g, 67 %). ¹H (CDCl₃): 1.40 (t, 3H, J = 7.0); 4.18 (q, 2H, J = 7.0); 5.85 (s, 1H); 6.90 (s(1), 1H). ¹³C (CDCl₃): 14.7; 65.7; 83.3; 98.4; 162.4. HRMS: Calcd. for C₅H₇IN₂IO + H : 238.9681. Found: m/z, 238.9621.

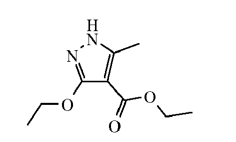

Ethyl 3-ethoxy-5-methyl-1H-pyrazole-4-carboxylate (**14**): This compound was obtained from diethyl 2-(1-ethoxyethylidene)malonate,¹⁴ using the protocol described above, as a solid in a 17 % yield after a chromatography over silica gel (dichloromethane / ethanol 94/6). ¹H (CDCl₃): 1.35 (t, 3H, J = 7.1); 1.43 (m, 3H); 2.47 (s, 3H); 4.30 (m, 4H); 9.2 (s, 1H). ¹³C (CDCl₃): 12.5; 14.3; 14.6; 59.7; 64.9; 97.2; 145.5; 163.0; 163.6. Calcd. for C₉H₁₄N₂O₃ + Na : 221.0902. Found: m/z, 221.0894.

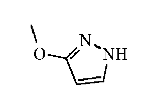

3-Methoxy-1H-pyrazole (**15**). Methyl 3-methoxyacrylate (**4**) (13.36 g, 0.114 mol) and hydrazine hydrochloride (8.08 g, 0.117 mol)) were refluxed in methanol (200 mL) for 48 hours. The solvent were removed under reduced pressure, the residue was dispersed in water (500 mL) and slowly made basic by the addition of solid sodium hydrogencarbonate. The aqueous phase was saturated with salt, extracted with dichloromethane; this organic phase was washed with a 1 N solution of sodium hydrogencarbonate three times, dried over sodium sulfate and concentrated to dryness. The residue was boiled in water (50 mL) in the presence of potassium hydroxide (3.8 g) for 6 hours. This was extracted with ethyl acetate; the organic phase was washed with brine dried over sodium sulfate and concentrated to dryness to yield compound **15** as an oil (0.85 g, 7.5 %). ¹H (CDCl₃): 3.92 (s, 3H); 5.73 (d, 1H, J = 2.5); 7.37 (d, 1H, J = 2.5). ¹³C (CDCl₃): 56.6; 89.6; 130.4; 164.4.

### Preparation of compounds 16a-b, 17a-b and 18a-b

Diethylethoxymethylenemalonate (0.0172 mol) and the relevant hydrazine hydrochloride derivative (0.0178 mol) were refluxed in ethanol (50 mL) for 16 hours. The solvent were removed under reduced pressure, the residue was dispersed in water (50 ml) and slowly made basic by the addition of solid sodium hydrogencarbonate. The aqueous phase was extracted with dichloromethane; this organic phase was washed with a 1 N solution of sodium hydrogencarbonate three times, dried over sodium sulfate and concentrated to dryness before a relevant purification if necessary as described below.

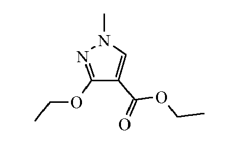

Ethyl 3-ethoxy-1-methyl-1H-pyrazole-4-carboxylate (**16a**): Obtained as an oil in a 17 % yield that crystallizes after a chromatography over neutral alumina containing 1.5 % water (cyclohexane / ethyl acetate 95/5 to 4/1). ¹H (CDCl₃): 1.27 (t, 3H, J = 7.1); 1.41 (t, 3H, J = 7.1); 3.72 (s, 3H); 4.24 (q, 2H, J = 7.1); 4.28 (q, 2H, J = 7.1); 7.63 (s, 1H). ¹³C (CDCl₃): 14.4; 14.7; 39.3; 59.7; 65.1; 99.3; 134.9; 162.1; 162.5. Calcd. for C₉H₁₄N₂O₃ + H : 199.1083. Found: m/z, 199.1046.

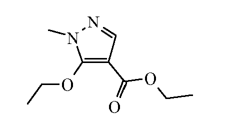

Ethyl 5-ethoxy-1-methyl-1H-pyrazole-4-carboxylate (**16b**): Obtained as an oil in a 17 % yield after a chromatography over neutral alumina containing 1.5 % water (cyclohexane /ethyl acetate 95/5 to 4/1). ¹H (CDCl₃): 1.35 (t, 3H, J = 7.1); 1.41 (t, 3H, J = 7.0); 3.68 (s, 3H); 4.27 (q, 2H, J = 7.1); 4.48 (q, 2H, J = 7.0); 7.76 (s, 1H). ¹³C (CDCl₃): 14.4; 15.4; 34.1; 59.9; 71.6; 99.3; 140.7; 154.7; 162.4. Calcd. for C₉H₁₄N₂O₃ + H : 199.1083. Found: m/z, 199.1001.

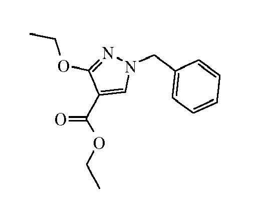

Ethyl 1-benzyl-3-ethoxy-1H-pyrazole-4-carboxylate (**17a**): Obtained as an oil in a 5.7 % yield after a chromatography over neutral alumina containing 1.5 % water (cyclohexane /ethyl acetate 97.5/2.5 to 95/5). ¹H (CDCl₃): 1.31 (t, 3H, J = 7.1); 1.44 (t, 3H, J = 7.1); 4.23 (q, 2H, J = 7.1); 4.33 (q, 2H, J = 7.1); 5.11 (s, 2H); 7.23 (m, 1H); 7.35 (m, 3H); 7.64 (s, 1H). ¹³C (CDCl₃): 14.4; 14.7; 56.2; 59.8; 65.2; 99.9; 127.9; 128.3; 128.9; 134.2; 135.3; 162.1; 162.6. Calcd. for C₁₅H₁₈N₂O₃ + Na : 297.1215. Found: m/z, 297.1219. 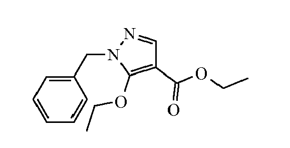 Ethyl 1-benzyl-5-ethoxy-1H-pyrazole-4-carboxylate (**17b**): Obtained as an oil in a 22 % yield after a chromatography over neutral alumina containing 1.5 % water (cyclohexane /ethyl acetate 97.5/2.5 to 95/5). ¹H (CDCl₃): 1.24 (m, 6H); 4.18 (q, 2H, J = 7.1); 4.31 (q, 2H, J = 7.1); 5.09 (s, 2H); 7.22 (m, 5H); 7.72 (s, 1H). ¹³C (CDCl₃): 14.4; 15.3; 51.1; 59.9; 71.7; 99.4; 127.5; 127.9; 128.7; 136.3; 141.2; 154.7; 162.4. Calcd. for C₁₅H₁₈N₂O₃ + H : 275.1396. Found: m/z, 275.1345.

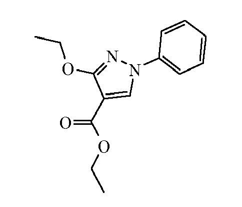

Ethyl 3-ethoxy-1-phenyl-1H-pyrazole-4-carboxylate (**18a**): Obtained as crystals (cyclohexane) in a 1.9 % yield after a chromatography over neutral alumina containing 1.5 % water (cyclohexane / ethyl acetate 97.5/2.5 to 95/5). ¹H (CDCl₃): 1.38 (t, 3H, J = 7.1); 1.51 (t, 3H, J = 7.1); 4.33 (q, 2H, J = 7.1); 4.46 (q, 2H, J = 7.1); 7.30 (m, 1H); 7.46 (m, 2H); 7.65 (m, 2H) ; 8.25 (s, 1H). ¹³C (CDCl₃): 14.4; 14.7; 60.1; 65.4; 102.2; 118.5; 126.6; 129.5; 131.1; 139.3; 162.5; 162.7. HRMS: Calcd. for C₁₄H₁₆N₂O₃ + H : 261.1239. Found: m/z, 261.1275.

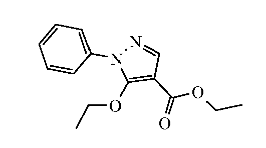

Ethyl 5-ethoxy-1-phenyl-1H-pyrazole-4-carboxylate (**18b**): Obtained as an oil in a 22 % yield after a chromatography over neutral alumina containing 1.5 % water (cyclohexane /ethyl acetate 97.5/2.5 to 95/5). Obtained as an oil ¹H (CDCl₃): 1.31 (t, 3H, J = 7.1); 1.37 (t, 3H, J = 7.1); 4.32 (q, 2H, J = 7.1); 4.42 (q, 2H, J = 7.1); 7.34 (m, 1H); 7.46 (m, 2H); 7.67 (m, 2H); 7.94 (s, 1H). ¹³C (CDCl₃): 15.7; 16.5; 61.4; 73.4; 102.3; 124.5; 128.8; 130.2; 139.2; 143.2; 156.1; 163.6. HRMS: Calcd. for C₁₄H₁₆N₂O₃ + H: 261.1239. Found: m/z, 261.1272.

### Results

As shown in scheme 1, the readily O-protected ethyl 3-ethoxy-1H-pyrazole-4-carboxylate (**8**) was prepared in one step from diethyl 2-(ethoxymethylene)malonate (**7**). This is achieved in a 41 % yield by the use of hydrazine hydrochloride in boiling ethanol. The other reaction product is the known ethyl 3-hydroxy-1H-pyrazole-4-carboxylate **9**, which is the sole reaction product between hydrazine hydrate and **7.**¹¹, ⁸ Surprisingly, this synthetic pathway has no precedent. This reaction turns out to also proceed in a similar yield when starting using hydrazine dihydrochloride and diethyl 2-((dimethylamino)methylene) malonate (**6**), which is easily prepared from diethyl malonate **(5).**¹³ On the other hand, lesser amount of 3-ethoxypyrazole (**10**) were obtained from ethyl 3-(dimethylamino)acrylate or ethylpropiolate. In any case, an acid hydrolysis of the carboxylate moiety of compound **8** also leads to its decarboxylation and thus, in a one pot process, to compound **10** in a 38 % yield from **7**. From compound **8** and **10**, further transformations allowed the preparation of the two iodo-bearing derivatives **11** and **12**. If the synthesis of compound **11** was trouble-free, many attempts were made to prepare the 5-halogenated alkoxypyrazole **12a.** Eventually, compound **12** was prepared from **8** in a 60 % yield using N-iodosuccinimide in boiling cyclohexane. The 5-brominated homolog **12b** was also obtained, from N-bromosuccinimide, in a 47 % yield. Moreover, under acid condition, compound **12a** led to 67 % of the 5-iodo-3-ethoxypyrazole (**13**). Other salts of hydrazine were tried (hydrobromide) giving the same yields.

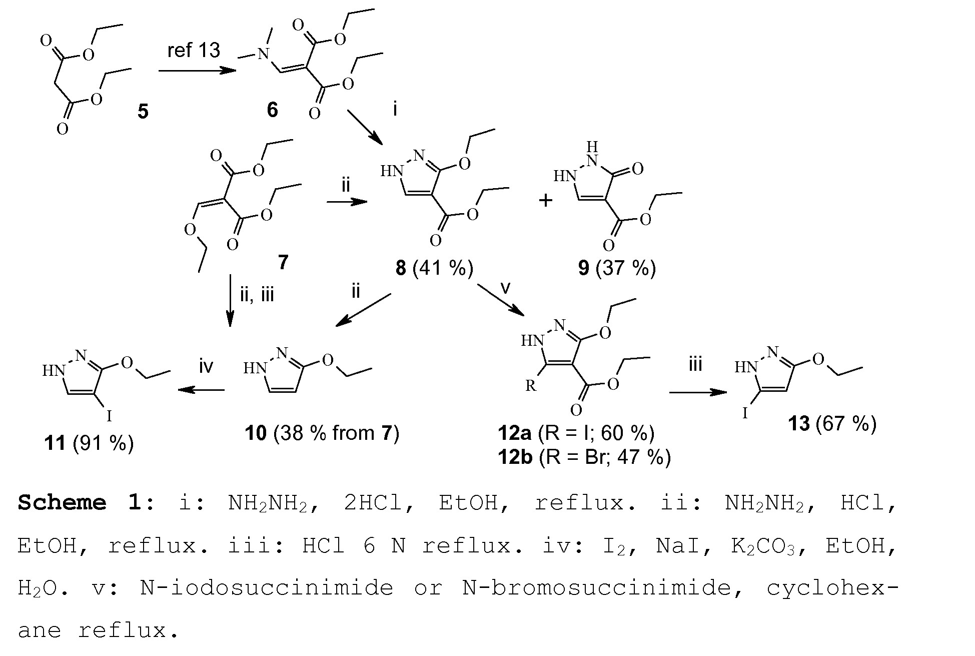

As shown in scheme 2, from the methyl homolog of compound **7,**¹⁴ the same reaction gave 17 % of the 5-methyl 3-alkoxy pyrazole **14.** Moreover, the condensation of hydrazine hydrochloride with methyl 3-methoxyacrylate led to the 3-methoxypyrazole **15** although in a lesser 7.5 % yield.

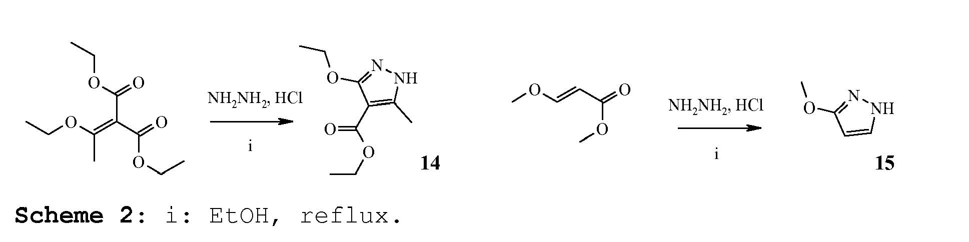

Further work led to the study of the condensation reaction between diethyl 2-(ethoxymethylene)malonate (**7**) and methyl, benzyl or phenyl hydrazine hydrochloride. As shown in scheme 3, these reactions led, aside from the expected isomeric pyrazolones, to substantial amount of the isomeric 3-ethoxy pyrazoles which could be separated. Their structure assignement was achieved either by long distance ¹H, ¹³C NMR correlation experiments or, for compounds **18a** and **18b** by further chemical transformations as described below.

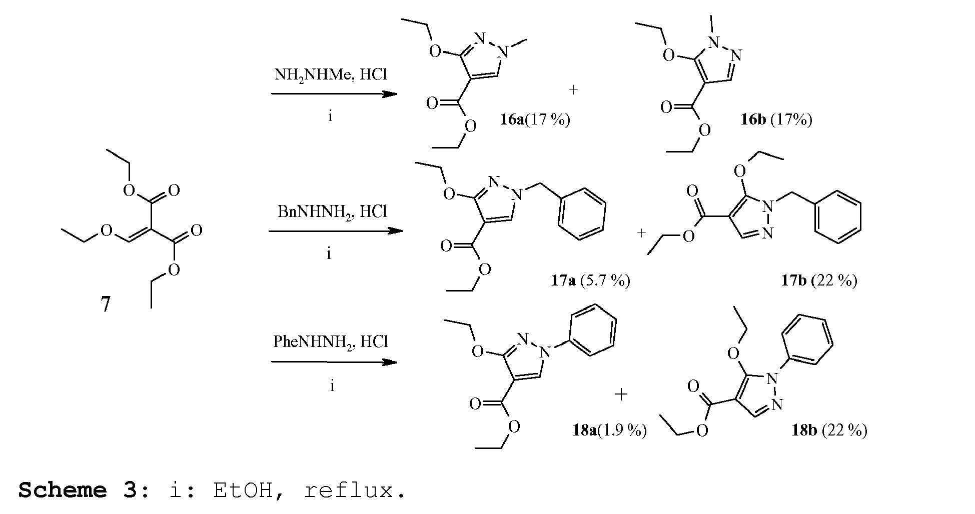

### Example 2: N-arylation study from compounds 8, 10-13

The pyrazole derivative (5.43 mmol), benzeneboronic acid (0.73 g, 5.97 mmol), pyridine (0.88 ml, 10.9 mmol, dried over 4Å molecular sieves), 4Å molecular sieves (2 g) and copper (II) acetate hydrate (1.62 g, 8.15 mmol) were dispersed in dichloromethane (100 mL). The suspension was stirred in open air for 48 hours. After concentration to dryness, the residue was absorbed on a small amount of silica gel and purified as described below for each pair of isomers.

From compound **8**, a chromatography as over neutral alumina containing 1.5 % water (cyclohexane - dichloromethane 8/1) led in this order to compound **18b** and then compound **18a** (92 %). The fraction containing **18b** had to be further purified by a second chromatography over neutral alumina containing 1.5 % water (cyclohexane - ethylacetate 9/1) to yield pure **18b** (7 %).
Ethyl 3-ethoxy-1-phenyl-1H-pyrazole-4-carboxylate (**18a**): as described above
Ethyl 5-ethoxy-1-phenyl-1H-pyrazole-4-carboxylate (**18b**): as described above.

From compound **11,** a chromatography over silica gel (cyclohexane - dichloromethane 8/2) led, in that order, to compound **19a** (71 %) and its isomer **19b** (1.5 %).

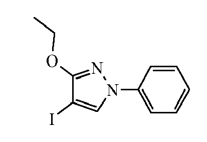

3-Ethoxy-4-iodo-1-phenyl-1H-pyrazole (**19a**): Obtained as an oil. 1.48 (t, 3H, J = 7.0); 4.42 (q, 2H, J = 7.0); 7.24 (m, 1H); 7.43 (m, 2H); 7.58 (m, 2H); 7.79 (s, 1H). ¹³C (CDCl₃): 14.7; 47.0; 65.6; 117.7; 125.7; 129.4; 131.8; 139.8; 163.7. HRMS: Calcd. for C₁₁H₁₁N₂OI + H : 314.9995. Found: m/z, 315.0037.

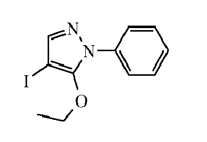

5-ethoxy-4-iodo-1-phenyl-1H-pyrazole (**19b**): Obtained as an oil. 1.32 (t, 3H, J = 7.0); 4.21 (q, 2H, J = 7.0); 7.34 (m, 1H); 7.47 (m, 2H); 7.54 (s, 1H); 7.68 (m, 2H). ¹³C (CDCl₃): 15.3; 45.3; 71.2; 122.4; 127.2; 129.0; 138.4; 144.2; 153.2. HRMS: Calcd. for C₁₁H₁₁N₂OI + H : 314.9995. Found: m/z, 315.0053.

From compound **12a,** a chromatography over neutral alumina containing 1.5 % water (cyclohexane - ethyl acetate 96/4) led in this order to compound **20b** (26 %) and then compound **20a** (60 %).

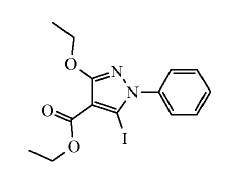

Ethyl 5-iodo-3-ethoxy-1-phenyl-1H-pyrazole-4-carboxylate (**20a**): Obtained as a solid. 1.40 (t, 3H, J = 7.0); 1.45 (t, 3H, J = 7.0); 4.37 (m, 4H); 7.48 (m, 5H). ¹³C (CDCl₃): 14.2; 14.6; 60.3; 65.1; 90.2; 105.4; 127.1; 128.9; 129.1; 140.1; 161.9; 163.8. HRMS: Calcd. for C₁₄H₁₅N₂O₃I + Na : 409.0025. Found: m/z, 409.0036.

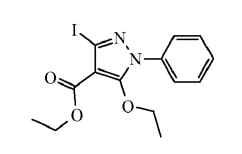

Ethyl 3-iodo-5-ethoxy-1-phenyl-1H-pyrazole-4-carboxylate (**20b**): Obtained as an oil. 1.28 (t, 3H, J = 7.1); 1.43 (t, 3H, J = 7.1); 4.27 (q, 2H, J = 7.1); 4.38 (q, 2H, J = 7.1); 7.39 (m, 1H) ; 7.46 (m, 2H); 7.65 (m, 2H). ¹³C (CDCl₃): 14.3; 15.2; 60.5; 72.7; 99.9; 104.7; 123.2; 128.3; 128.8; 137.1; 154.7; 161.2. HRMS: Calcd. for C₁₄H₁ₛN₂O₃I + Na : 409.0025. Found: m/z, 315.0026.

From compound **12b,** a chromatography over neutral alumina containing 1.5 % water (cyclohexane - ethyl acetate 97/3) led, in that order, to compound **21b** (13 %) and then its isomer **21a** (54 %).

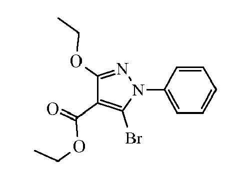

Ethyl 5-bromo-3-ethoxy-1-phenyl-1H-pyrazole-4-carboxylate (**21a**): Obtained as an oil. 1.37 (t, 3H, J = 7.0); 1.46 (t, 3H, J = 7.0); 4.37 (m, 4H); 7.46 (m, 5H). ¹³C (CDCl₃): 14.3; 14.6; 60.4; 65.0; 101.3; 118.4; 126.2; 128.9; 129.0; 138.5; 161.8; 162.7. HRMS: Calcd. for C₁₄H₁₅N₂O₃79Br + Na : 361.0164. Found: m/z, 361.0184.

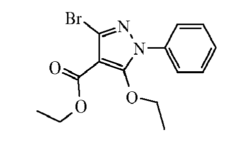

Ethyl 3-bromo-5-ethoxy-1-phenyl-1H-pyrazole-4-carboxylate (**21b**): Obtained as an oil. ¹H (CDCl₃) : 1.29 (t, 3H, J = 7.0); 1.44 (t, 3H, J = 7.0); 4.28 (q, 2H, J = 7.0); 4.38 (q, 2H, J = 7.0); 7.37 (m, 1H); 7.46 (m, 2H); 7.66 (m, 2H). ¹³C (CDCl₃): 14.3; 15.2; 60.6; 72.8; 101.4; 123.2; 128.1; 128.8; 129.1; 137.1; 155.1; 161.2. HRMS: Calcd. for C₁₄H₁₅N₂O₃⁷⁹Br + Na : 361.0164. Found: m/z, 361.0172.

From compound **10,** a chromatography over neutral alumina containing 1.5 % water (cyclohexane - dichloromethane 9/1 and then 2/1) led, in that order, to compound **22a** (56 %) and its isomer **22b** (20 %).

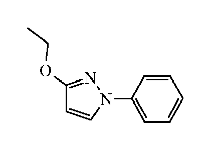

Ethyl 3-ethoxy-1-phenyl-1H-pyrazole (**22a**): Obtained as an oil. ¹H (CDCl₃): 1.46 (t, 3H, J = 7.0); 4.33 (q, 2H, J = 7.0); 5.90 (d, 1H, J = 2.5); 7.23 (m, 1H); 7.42 (m, 2H); 7.63 (m, 2H); 7.74 (d, 1H, J = 2.5) . ¹³C (CDCl₃): 14.8; 64.9; 93.7; 117.8; 125.2; 125.5; 129.3; 140.2; 164.5. HRMS Calcd. for C₁₁H₁₂N₂O + H : 189.1028. Found: m/z, 189.1061.

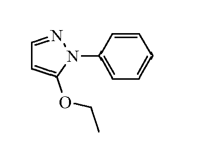

Ethyl 5-ethoxy-1-phenyl-1H-pyrazole (**22b**): Obtained as an oil. ¹H (CDCl₃) : 1.47 (t, 3H, J = 7.1); 4.19 (q, 2H, J = 7.1); 5.67 (d, 1H, J = 1.7); 7.28 (m, 1H); 7.42 (m, 2H); 7.51 (d, 1H, J = 1.7); 7.77 (m, 2H) . ¹³C (CDCl₃) : 14.6; 67.9; 86.2; 122.0; 126.2; 128.8; 138.8; 139.6; 154.6. HRMS: Calcd. for C₁₁H₁₂N₂O + H : 189.1028. Found: m/z, 189.0971.

Acid hydrolysis of compounds **18a** and **18b:** In a 60 mL roundbottomed thick glass tube fitted with a PTFE-faced screwcap, a mixture of the considered pyrazole (1 mmol) and 33 % hydrogen bromide in acetic acid (1.5 mL) was heated at 140 °C for 4 hours. The resulting solution was cooled, diluted in water extracted with ethyl acetate. The organic layer was washed with brine, dried over sodium sulfate and concentrated to dryness. The resulting residues were purified in both cases by a chromatography over silica gel (dichloromethane-ethanol 99/1) to yield compound **23a** or **23b** as described below.

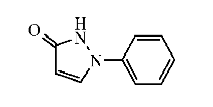

1-phenyl-1H-pyrazol-3(2H)-one (**23a**): Obtained as a white powder in an 81 % yield. ¹H (DMSO-*d₆*): 5.80 (d, 1H, J = 2.5); 7.18 (m, 1H); 7.47 (m, 2H); 7.67 (m, 2H); 8.20 (d, 1H, J = 2.5); 10.18 (s(br), 1H). ¹³C (DMSO-*d₆*) : 94.3; 116.7; 124.5; 128.3; 129.3; 139.8; 162.6. HRMS: Calcd. for C₉H₈N₂O + H : 161.0715. Found: m/z, 161.0793.

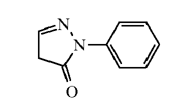

1-phenyl-1H-pyrazol-5(4H)-one (**23b**): Obtained as an oil which solidified in a 71 % yield. ¹H (CDCl₃): 3.51 (d, 2H, J = 1.2); 7.21 (m, 1H); 7.43 (m, 2H); 7.49 (t, 1H, J = 1.2); 7.88 (m, 2H). ¹³C (CDCl₃): 40.9; 119.0; 125.4; 128.9; 137.9; 146.8; 169.9. HRMS: Calcd. for C₉H₈N₂O + H : 161.0715. Found: m/z, 161.0799.

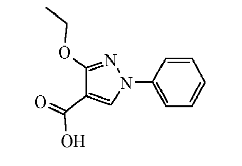

3-Ethoxy-1-phenyl-1H-pyrazole-4-carboxylic acid (**24**): ester **18a** (0.52 g, 2 mmol) was refluxed in a 1/1 water - ethanol solution (20 mL) containing sodium hydroxide (0.3 g, 3.8 mmol) for one hour. The resulting solution was diluted in water, made acid with 2N hydrochloric acid and the precipitate was filtered, washed with water and dried under vacuum to yield compound **24** as a white powder (0.39 g, 85 %). ¹H (DMSO-*d₆*): 1.37 (t, 3H, J = 7.1); 4.33 (q, 2H, J = 7.1); 7.32 (m, 1H); 7.48 (m, 2H); 7.85 (m, 2H); 8.20 (s, 1H). ¹³C (DMSO-*d₆*): 15.0; 65.1; 102.6; 118.4; 126.8; 129.9; 133.2; 139.3; 162.4; 163.3. HRMS: Calcd. for C₁₂H₁₂N₂O₃ + H : 233.0926. Found: m/z, 233.0973.

### Results:

Scheme 4 depicts the copper-based N-phenylation of these compounds using phenylboronic acid. ¹⁵⁻¹⁸ From compound **8,** a 92 % yield of the N-1 phenylation product **18a** along with 7 % of its isomer **18b** were isolated. The latter isomer is actually the only one which had been partially reported previously.^{19, 20} The N-phenylation of the 4-iodo bearing compound **11** gave 71 % of the N1-phenylation product **19a** and only traces of **19b.** On the other hand, this reaction proceeded with a lesser selectivity from compound **12a** as compound **20a** was obtained in a 60 % yield along with 26 % of 20b. A similar result was observed from **12b** which led to compound **21a** and **21b** in 54 and 13 % yield respectively. And, from compound **10,** the isomers **22a** and **22b** are obtained in 56 and 20 % yield respectively. Arylation of compound **13** also proceeded well; however, despite many trials, we could not separate the 1/3 mixture of the corresponding isomeric products.

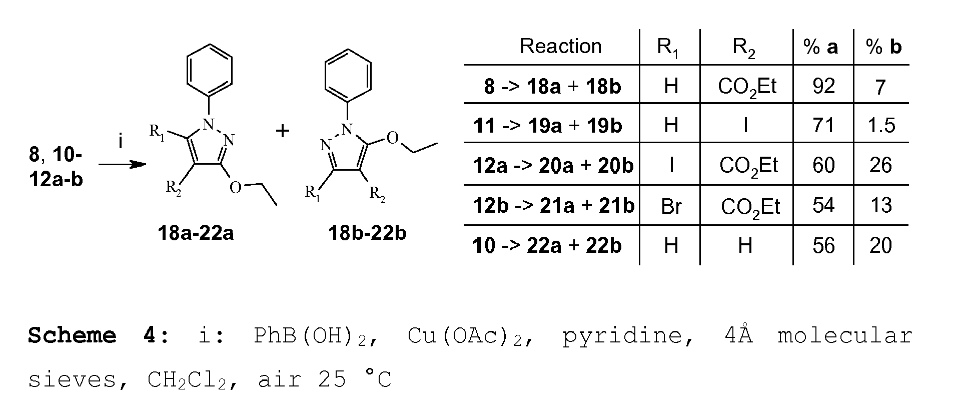

As long distance correlation or NOE-based NMR experiments are of little use to determine the regioselectivity of these N-phenylation reactions, this was ascertained beyond any doubt by the following transformations of the separated isomers. As depicted in scheme 5, an exhaustive acid hydrolysis of **18a** and **18b** led to compounds **23a** and **23b.** The ¹H and ¹³C NMR spectra of **23b** in deuterated chloroform pointed out the occurrence of a methylene component only compatible with the mesomer **23b** drawn in scheme 2. The saponification of the ester function of compound **18a** gave the acid **24** which upon heating in the presence of an equivalent amount of methanesulfonic gave 56 % of decarboxylation product corresponding to the isomer **22a.** A partial (as the evolving hydrogen iodide poisons the catalyst) palladium-catalysed reduction of compound **19a** also gave small amount of the isomer **22a.** Under these reduction conditions, the bromine-bearing compound **21a** led to the isomer **18a.** Moreover, as it is shown below, further chemistry using compound **20a** and **21a** gave the same reaction product thus completing the structure assignment of all the N-phenyl derivatives.

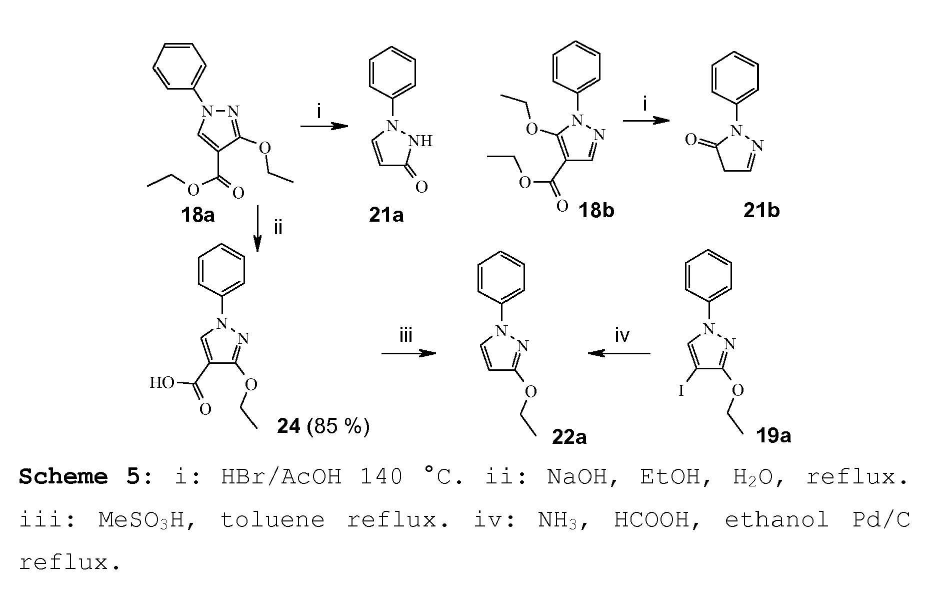

### Example 3: C4 and C5-phenylation of the N-arylated compounds of Example 2 or of the 5-haloderivatives compounds of Example 1

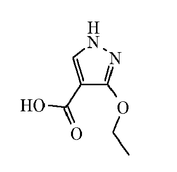

3-Ethoxy-1H-pyrazole-4-carboxylic acid (**25**): compound **8** (1 g, 5.43 mmol) was heated to reflux in 1N sodium hydroxide solution (11 mL) for one hour (a shorter time is likely to be better) and upon acidification the precipitate was filtered, washed with water and dried under vacuum to yield **25** as a white powder (0.49 g, 58 %). ¹H (DMSO-*d₆*): 1.30 (t, 3H, J = 7.0); 4.20 (q, 2H, J = 7.0); 7.98 (s, 1H); 11.8 (s (1), 1H); 12.5 (s (1), 1H). ¹³C (DMSO-*d₆*): 14.6; 64.0; 98.5; 134.3; 161.4; 163.4. HRMS: Calcd. for C₆H₈N₂O₃ + H : 157.0613. Found: m/z, 157.0600.

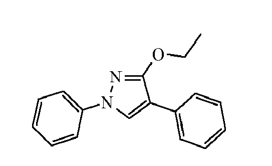

3-Ethoxy-1,4-diphenyl-1H-pyrazole (**26**): In a 10 mL biotage-adapted tube, compound **24** (0.11 g; 0.47 mmol), bromobenzene (0.068 mL; 0.64 mmol), potassium carbonate (0.078 g; 0.57 mmol) copper iodide (0.009 g; 0.047 mmol), phenanthroline (0.013 mg; 0.071 mmol) and 4A molecular sieves (0.02 g) were dispersed in N-methylpyrolidinone (4 mL). The oxygen was removed by a slow stream of argon and palladium acetate (5 mg, 0.023 mmol) was then added before sealing the tube. This was heated in the microwave oven for 40 minutes at 210 °C. The resulting suspension was concentrated to dryness under high vacuum. The residue was dispersed in water and extracted with ethyl acetate. The organic phase was washed with water, dried over sodium sulfate and concentrated to dryness. The residue was purified by a chromatography over silica gel (cyclohexane /dichloromethane 95-5) to yield compound **26** as a solid (0.010 g; 8 %). ¹H (CDCl₃): 1.58 (t, 3H, J = 7.0); 4.55 (q, 2H, J = 7.0); 7.27 (m, 2H); 7.48 (m, 4H); 7.70 (m, 2H); 7.81 (m, 2H); 8.04 (s, 1H). ¹³C (CDCl₃): 15.0; 65.0; 109.9; 117.7; 124.4; 125.2; 126.1 (two signals); 128.6; 129.4; 131.8; 140.1; 161.4. HRMS: Calcd. for C₁₇H₁₆N₂O + H : 265.1341. Found: m/z, 265.1365.
Under these reaction conditions, the N-phenylated derivative **22a** described above was obtained in a 41 % yield from acid **25.**

**Representative aryl-aryl coupling procedure, preparation of compound 27.** In a 10 mL biotage-adapted tube, compound **12b** (0.09 g; 0.34 mmol), phenyl boronic acid (0.054 g; 4.42 mmol), cesium carbonate (0.28 g; 8.47 mmol) were dispersed in a mixture of propanol (3 mL) and water (2 mL). The oxygen was removed by a slow stream of argon and [1,1'-bis(diphenylphosphino)ferrocene] dichloropalladium complexed with dichloromethane (0.057 mmol) was then added before sealing the tube. This was heated in the microwave oven for 30 minutes at 120 °C. The resulting suspension was diluted in water, extracted with dichloromethane. The organic layer was washed with brine, dried over sodium sulfate and concentrated to dryness. The residue was purified by a chromatography over silica gel (dichloromethane /ethanol 99-1) to yield compound **27** as an oil (0.065 g; 73 %).

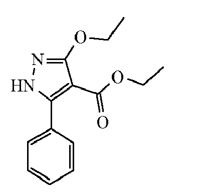

Ethyl 5-ethoxy-3-phenyl-1H-pyrazole-4-carboxylate (**27**): ¹H (CDCl₃): 1.17 (t, 3H, J = 7.0); 1.36 (t, 3H, J = 7.0); 4.16 (q, 2H, J = 7.0); 4.21 (q, 2H, J = 7.0); 7.38 (m, 3H); 7.51 (m, 2H); 10.74 (s(l), 1H). ¹³C (CDCl₃): 14.0; 14.6; 59.8; 65.0; 96.8; 128.1; 129.0; 129.3; 129.5; 147.3; 163.2; 163.3. HRMS: Calcd. for C₁₄H₁₆N₂O₃ + H : 261.1239. Found: m/z, 261.1228.

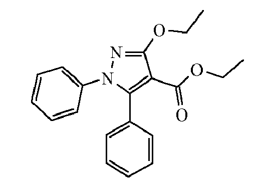

Ethyl 3-ethoxy-1,5-diphenyl-1H-pyrazole-4-carboxylate (**28**): This compound was obtained from compound **20a** (or **21a**) using the procedure described for the preparation of compound **27** as an oil that slowly solidifed in 75 % yield. ¹H (CDCl₃): 1.22 (t, 3H, J = 7.0); 1.31 (t, 3H, J = 7.0); 4.15 (q, 2H, J = 7.0); 4.48 (q, 2H, J = 7.0); 7.28 (m, 10H). ¹³C (CDCl₃): 13.9; 14.8; 59.7; 65.1; 100.1; 125.1; 127.2; 127.9; 128.7; 128.9; 129.8; 130.4; 139.2; 144.4; 162.7; 162.8. HRMS: Calcd. for C₂₀H₂₀N₂O₃ + H : 337.1552. Found: m/z, 337.1545.

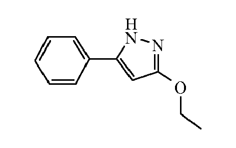

3-ethoxy-5-phenyl-1H-pyrazole **(29)**²¹ **:** Compound **27** (0.08 g, 0.30 mmol) and sodium hydroxide (1 g, 25 mmol) were heated to reflux in a mixture of water (4 mL) and ethanol (2 mL) for 12 hours. The solution was extracted with ethyl acetate, dried over sodium sulfate and concentrated to dryness to yield compound **29** (0.05 g, 86 %) featuring analytical data similar to the reported one.²¹

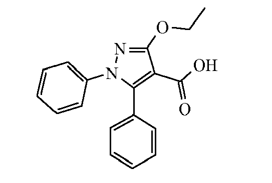

3-Ethoxy-1,5-diphenyl-1H-pyrazole-4-carboxylic acid (**30**): The protocol used for the preparation of compound **24** was used from **28** to yield compound **30** as a white powder (94 % yield). ¹H (DMSO-*d₆*): 1.38 (t, 3H, J = 7.1); 4.32 (q, 2H, J = 7.1); 7.15 (m, 2H); 7.30 (m, 8H). ¹³C (DMSO-*d₆*): 14.7; 64.3; 99.8; 125.4; 127.5; 127.8; 128.7; 128.8; 129.4; 130.3; 138.8; 146.3; 161.7; 163.0. HRMS: Calcd. for C₁₈H₁₆N₂O₃ + H : 309.1239. Found: m/z, 309.1239.

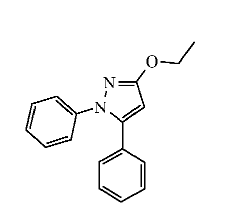

3-ethoxy-1,5-diphenyl-1H-pyrazole **(31)**²¹: Acid **30** (0.08g, 0.25 mmol) and methanesulfonic acid (0.016 mL, 0.25 mmol) were refluxed in toluene (20 mL) for two hours. The solution was washed with a saturated sodium hydrogencarbonate solution dried over sodium sulfate and concentrated to dryness to yield compound **31** as an oil (0.05 g; 73 %). ¹H (CDCl₃) : 1.46 (t, 3H, J = 7.1); 4.34 (q, 2H, J = 7.1); 5.97 (s, 1H); 7.27 (m, 10H). ¹³C (CDCl₃) : 14.9; 64.7; 93.7; 124.9; 126.7; 128.3; 128.4; 128.7; 128.8; 130.7; 140.1; 144.1; 163.5. HRMS: Calcd. for C₁₇H₁₆N₂O + H : 265.1341. Found: m/z, 265.1286.

### Results:

From acid **25,** readily obtained by the saponification of compound **8,** the N-phenylated compound **22a,** resulting from an Ullmann/Buchwald-Hartwig reaction,²² was the only product isolated (in a 41 % yield). From acid **24,** the expected C-4 arylation product **26,** resulting form a decarboxylative coupling reaction, was obtained; although in only an 8 % yield. A far better 72 % yield of **26** was obtained from the 4-iodinated isomer **19a** using the more conventional Suzuki-Miyaura aryl-aryl cross coupling reaction. The duration of this coupling reaction was greatly shorten by the use of the [1,1'-bis(diphenylphosphino)ferrocene] dichloropalladium as a precatalyst^{23, 24} as well as the recourse to a microwave oven. The use of these aryl-aryl coupling conditions successfully led to the C-5 phenyl derivatives **27** or **28,** from compound **12a** or **20a** in 75 and 74 % yield respectively. Very similar results were obtained from the 5-bromo homologues **12b** or **21a** (giving 73 or 62 % yield of compound **27** or **28** respectively). However, attempts to achieve a direct C-4 arylation of compound **11** only led to extensive decomposition. Even the addition of lithium chloride as described in previously reported palladium-based aryl coupling reaction of heterocycles featuring an NH component did not help.²⁵⁻²⁸ To complete this synthetic exploration, the removal of the carboxyl moiety of compounds **27** and **28** was also undertook. In the case of compound **27,** this was achieved using strongly basic condition leading to the known²¹ compound **29** in 86 % yield. From ester **28,** a two-step sequence was devised leading first to the acid **30** which undertook an acid-catalysed decarboxylation leading to the known^{21, 9} compound **31** in a 68 % overall yield.

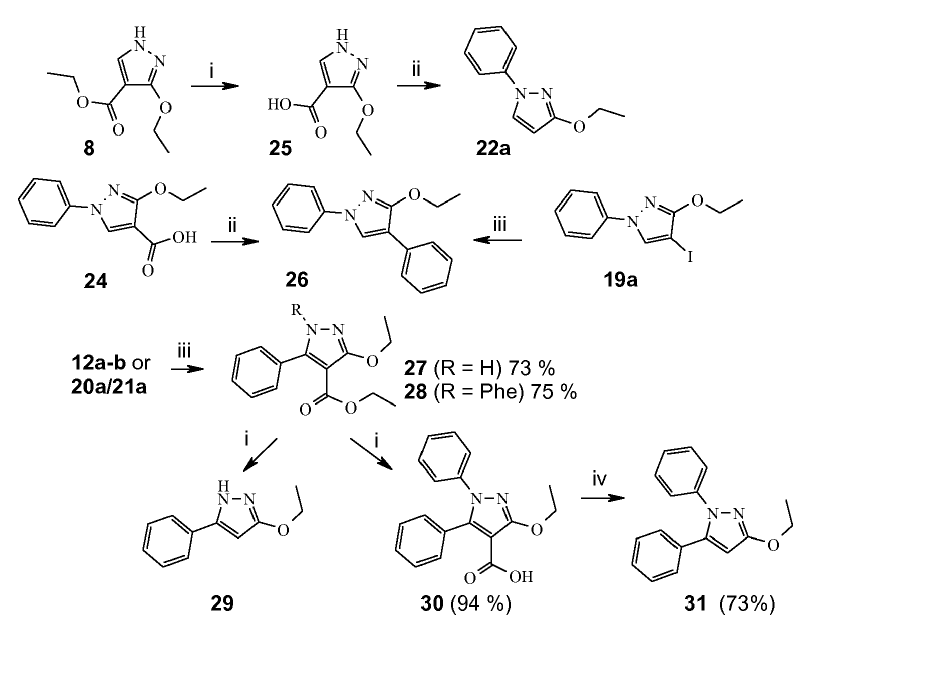

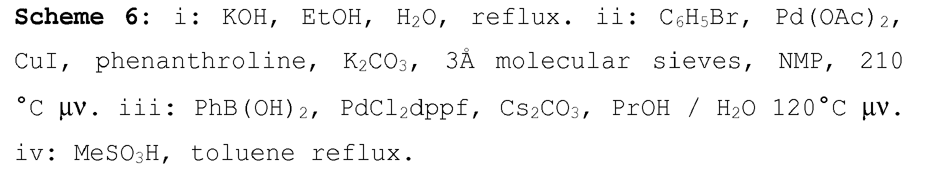

### Example 4: C4- arylation, benzylation and phenethylation and C5-benzylation of derivatives of and of compounds of Example 1

### Preparation of the mixture of mesyl-protected pyrazoles

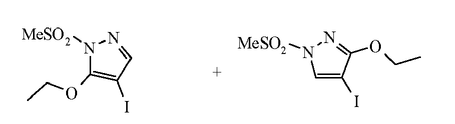

5-ethoxy-4-iodo-1-(methylsulfonyl)-1H-pyrazole and 3-ethoxy-4-iodo-1-(methylsulfonyl)-1H-pyrazole **(32a-b)**: compound **11** (2.06 g, 8.65 mmol) was dissolved in ethyl acetate (150 mL), triethylamine (1.9 mL, 13.8 mmol; dried over 4Å molecular sieves) was added followed by mesylchloride (1.0 mL, 13.0 mmol). The solution was protected from moisture by a calcium chloride guard and stirred for two hours at room temperature. This was washed with 2 N potassium carbonate, 0.5 N hydrochloric acid and brine, dried over sodium sulfate and concentrated to dryness to yield the 2/5 mixture of the two isomers as an oil that slowly crystallized (2.68 g, 98 %). ¹H (CDCl₃): 1.45 (d, 5/7 of 3H, J = 7.1); 1.49 (d, 2/7 of 3H, J = 7.1); 3.25 (s, 5/7 of 3H); 3.31 (s, 2/7 of 3H); 4.41 (m, 2H); 7.61 (s, 2/7 of 1H) ; 7.90 (s, 5/7 of 1H). *m*/*z* (LC/MS) = 253.

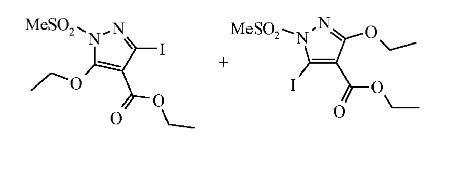

ethyl 3-ethoxy-5-iodo-1-(methylsulfonyl)-1H-pyrazole-4-carboxylate and ethyl 5-ethoxy-3-iodo-1-(methylsulfonyl)-1H-pyrazole-4-carboxylate **(33a-b):** From compound **12a**, the protocol described above led to a 1/1 mixture of the possible isomers as a solid (0.46 g, 89 %). ¹H (CDCl₃): 1.45 (m, 6H); 3.37 (s, 1/2 of 3H); 3.40 (s, 1/2 of 3H); 4.39 (m, 3/4 of 4H); 4.49 (q, 1/4 of 4H, J = 7.1).

### General procedure for the C-4 arylation of the mesyl-protected pyrazoles 32a-b.

In a 10 mL biotage-adapted tube, the mixture of the mesyl-protected pyrazoles (1.38 g, 4.37 mmol), the relevant boronic acid (5.68 mmol), cesium carbonate (3.56 g; 10.9 mmol) were dispersed in a mixture of propanol (3 mL) and water (2 mL). The oxygen was removed by a slow stream of argon and [1,1'-bis(diphenylphosphino)ferrocene] dichloropalladium complexed with dichloromethane (0.178 g, 0.218 mmol) was then added before sealing the tube. This was heated in the microwave oven for 40 minutes at 120 °C. This was diluted in water, extracted with ethyl acetate; the organic layer was washed with brine and dried over sodium sulfate and concentrated to dryness. The residue was dissolved in ethanol (150 mL) and potassium hydroxide (0.8 g) this was heated at 90 °C for two hours. This was diluted in water, made slightly acid with ammonium chloride, extracted with ethyl acetate, the organic layer was washed with brine and dried over sodium sulfate and concentrated to dryness. The residue was further purified as described below. 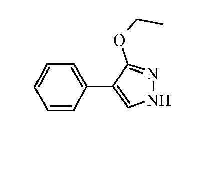 4-phenyl-3-ethoxy-1H-pyrazole (**34**): This compound was purified by a chromatography over neutral alumina containing 1.5 % of water (dichloromethane / ethanol 99/1) and the fraction was further purified by a recrystallization in cyclohexane to yield compound **34** (57 %) as a white powder. ¹H (CDCl₃): 1.49 (t, 3H, J = 7.0); 4.39 (q, 2H, J = 7.0); 7.23 (m, 1H); 7.38 (m, 2H); 7.66 (s, 1H); 7.68 (m, 2H); 9.16 (s (1), 1H). ¹³C (CDCl₃): 14.9; 64.7; 107.2; 125.8; 125.9; 127.0; 128.5; 131.9; 160.7. HRMS: Calcd. for C₁₁H₁₅N₂O + H : 189.1028. Found: m/z, 189.0984.

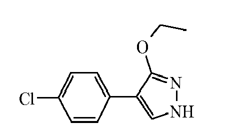

4-(4-chlorophenyl)-3-ethoxy-1H-pyrazole (**35**): This compound was purified by a chromatography over neutral alumina containing 1.5 % of water (dichloromethane / ethanol 99/1) to give compound **35** (23 %) as a white powder. ¹H (CDCl₃) : 1.47 (t, 3H, J = 7.0); 4.38 (q, 2H, J = 7.0); 7.33 (m, 2H); 7.61 (s, 2H); 7.63 (s, 1H). ¹³C (CDCl₃): 14.9; 64.8; 106.1; 127.0; 127.1; 128.9; 130.5; 131.3; 160.6. HRMS: Calcd. for C₁₁H₁₄N₂O³⁵Cl + H : 223.0638. Found: m/z, 223.0676.

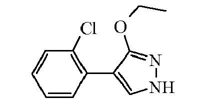

4-(2-chlorophenyl)-3-ethoxy-1H-pyrazole (**36**): This compound was purified by a chromatography over neutral alumina containing 1.5 % of water (dichloromethane / ethanol 99/1) to give compound **36** (33 %) as a white powder. ¹H (CDCl₃): 1.44 (t, 3H, J = 7.0); 4.37 (q, 2H, J = 7.0); 7.20 (m, 1H); 7.29 (m, 1H); 7.45 (s, 1H); 7.70 (m, 1H); 7.83 (s, 1H). ¹³C (CDCl₃): 14.9; 65.0; 103.7; 126.6; 127.5; 130.0; 130.3; 130.4; 131.0; 132.3; 160.8. m/z (LC/MS) = 223.

General procedure for the C-4 benzylation and phenethylation of **11** or C-5 benzylation of **13** and **33a-b.** In a biotage 20 mL tube, benzylzinc bromide or phenethyl zinc bromide (0.5 M in THF; 2.52 mmol) and THF (1 mL) were degassed with a slow stream of argon for ten minutes. The pyrazole halide (either **11, 13** or the mixture of **33a-b)** (0.84 mol) and [1,1'-bis(diphenylphosphino)ferrocene] dichloropalladium complexed with dichloromethane (0.042 mmol) were then added, and the tube was degassed again with a slow stream of argon for 5 min prior to capping. The reaction vessel was then heated at 85 °C for 4 hours in an oil bath. After cooling to room temperature, water and a 1N solution of potassium sodium tartrate (3 mL) were added. The aqueous layer was extracted with ethyl acetate. The combined organic layers were dried over sodium sulphate, filtered and concentrated to dryness. The residue was purified by chromatography over silica gel (dichloromethane - ethanol 99/1).

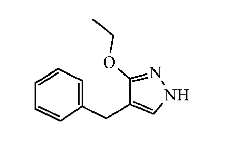

4-benzyl-3-ethoxy-1H-pyrazole (**37**): Obtained as an oil. ¹H (CDCl₃): 1.40 (t, 3H, J = 7.1); 3.74 (s, 2H); 4.28 (q, 2H, J = 7.1); 7.06 (s, 1H); 7.26 (m, 5H). ¹³C (CDCl₃) : 14.9; 28.4; 64.7; 105.3; 125.9; 128.3; 128.5; 128.7; 141.0; 161.4. Calcd. for C₁₂H₁₄N₂O + H : 203.1184. Found: m/z, 203.1122.

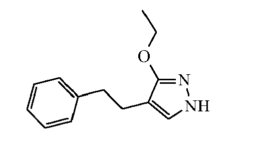

3-ethoxy-4-phenethyl-1H-pyrazole (**38**): Obtained as an oil. ¹H (CDCl₃): 1.42 (t, 3H, J = 7.1); 2.71 (m, 2H); 2.88 (m, 2H); 4.28 (q, 2H, J = 7.1); 7.04 (s, 1H); 7.20 (m, 3H); 7.30 (m, 2H). ¹³C (CDCl₃) : 14.9; 24.0; 35.9; 64.5; 105.3; 125.8; 128.1; 128.2; 128.5; 141.9; 161.7. m/z (LC/MS) = 217.

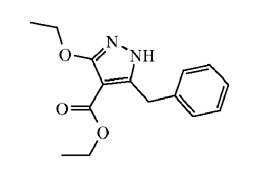

ethyl 5-benzyl-3-ethoxy-1H-pyrazole-4-carboxylate (**39a**) Obtained as an oil. ¹H (CDCl₃): 1.31 (t, 3H, J = 7.1); 1.34 (t, 3H, J = 7.1); 4.28 (m, 6H); 7.28 (m, 5H). ¹³C (CDCl₃): 14.3; 14.6; 32.7; 29.8; 64.9; 97.0; 127.1; 128.8; 128.9; 136.1; 147.5; 163.2; 163.5. m/z (LC/MS) 275.

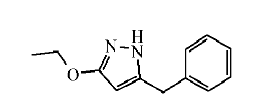

5-benzyl-3-ethoxy-1H-pyrazole (**39b**) Obtained as colorless crystals. ¹H (CDCl₃): 1.38 (t, 3H, J = 7.1); 3.96 (s, 2H); 4.17 (q, 2H, J = 7.1); 5.54 (s, 1H); 7.28 (m, 5H). ¹³C (CDCl₃): 14.8; 32.8; 65.0; 89.6; 126.9; 128.7; 128.8; 137.2; 144.5; 163.2. m/z (LC/MS) = 203.

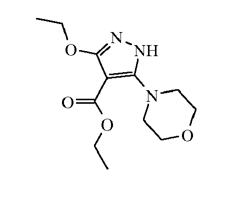

Ethyl 3-ethoxy-5-morpholino-1H-pyrazole-4-carboxylate (**40**): In a biotage 5 mL tube, ethyl 3-ethoxy-5-iodo-1H-pyrazole-4-carboxylate (0.2 g; 0.64 mmol) and morpholine (1.2 mL; 13.8 mmol) dissolved in toluene (5 mL). The reaction vessel was then heated at 170 °C for 4 hours using a microwave oven. This was concentrated to dryness and the residue was purified by chromatography on silica gel (dichloromethane - ethanol 98/2) to yield compound **40** as an oil (0.04 g; 23 %) . ¹H (CDCl₃): 1.33 (t, 3H, J = 7.1); 1.40 (t, 3H, J = 7.1); 3.28 (m, 4H); 3.83 (m, 4H); 4.25 (m, 4H); 7.90 (s(l), 1H). ¹³C (CDCl₃): 14.3; 14.7; 49.4; 59.7; 64.8; 66.3; 87.1; 154.2; 162.4; 163.00. Calcd. for C₁₂H₁₉N₃O₄ + Na : 292.1273. Found: m/z, 292.1220.

### Results

Despite some trials, the Suzuki-based aryl-aryl coupling reactions with the NH-bearing compound **11** were never very satisfactory. Depending on the catalyst used, some of these trials led to a mixture containing the expected compound as well as unreacted and reduced material. For this reason, we set to protect compound **11** using a mesyl group. The reaction of **11** with mesylchloride under basic conditions led to an isomeric mixture **32a-b** which was used whithout further purification in the aryl-aryl coupling step. This allowed us to prepare an array of 4-aryl compounds such as **34-36.** Interstingly the Negishi-beased carbon-carbon bond formation reaction turned out to be possible from the NH-bearing compound **11.** The use of benzyl or phenethyl zinc bromide allowed the preparation of compound **37** and **38.** On the other hand, preparation of the 5-benzyl derivative **39a** required the use of the N-protected mixture of compounds **33a-b** as little reaction was observed from the NH-bearing compound **12a.** On the other hand, from the unprotected compound **13,** the Negishi reaction with benzylzinc bromide gave 63 % of the 5-benzylderivative **39b.**

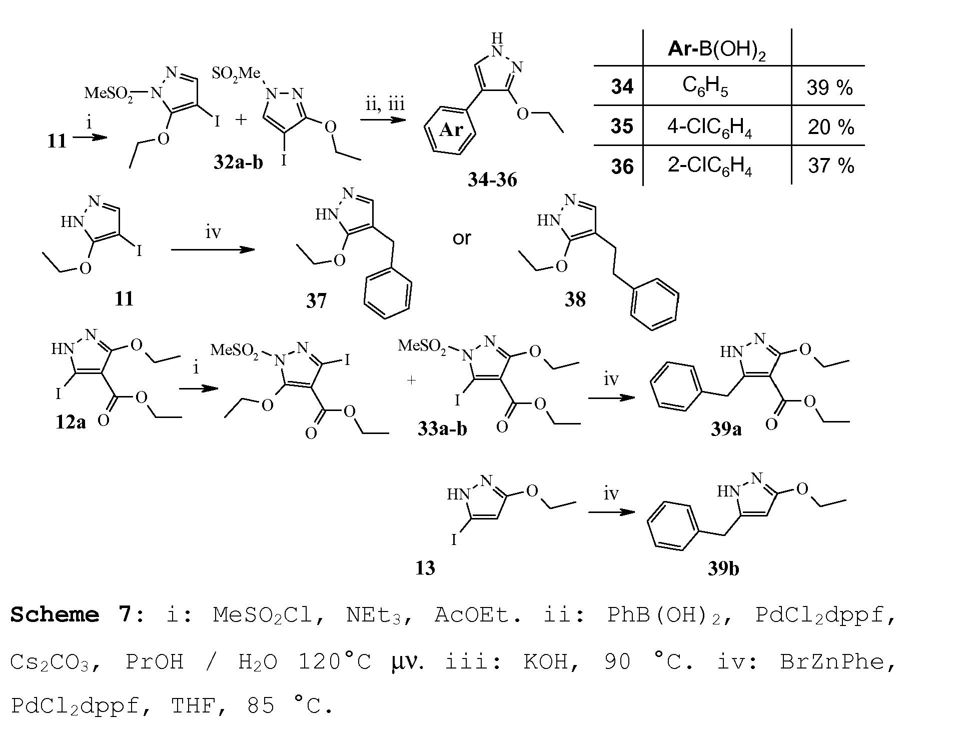

The difference of reactivity between compounds **11** and **12a** toward the Suzuki aryl-aryl coupling reaction may have its origin in the difference of lability of the iodine atom. Indeed, it was possible to prepare compound **40** by a nucleophilic substitution without the recourse to copper or palladium-based methods:^{29, 30} On the other hand, under these conditions, no reaction took place from compound **13** either.

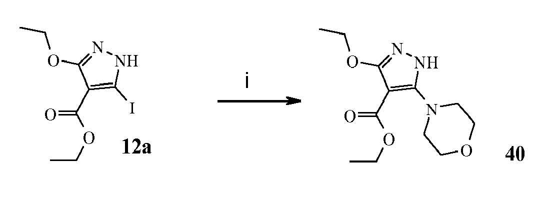

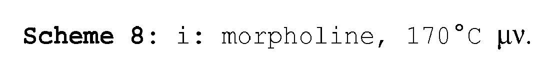

### Example 5: Halogenation of 4-arylpyrazoles 34 and 35 aryl-aryl coupling with the reaction products

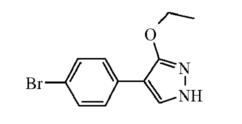

4-(4-bromophenyl)-3-ethoxy-1H-pyrazole (**41**): Compound **34** (0.1 g, 0.53 mmol) and bromine (0.031 mL, 0.63 mmol) were heated to reflux in cyclohexane (5 mL) for 2 hours. This was diluted in ethyl acetate washed with water, dried over sodium sulfate and concentrated to dryness. The residu was purified by chromatography over neutral alumina containing 1.5 % of water (dichloromethane / ethanol 99/1) to yield compound **41** as a white solid (0.07 g, 49 %). ¹H (CDCl₃): 1.49 (t, 3H, J = 7.0); 4.40 (q, 2H, J = 7.0); 7.48 (m, 2H); 7.56 (m, 2H); 7.68 (s, 1H). ¹³C (CDCl₃): 14.9; 65.2; 106.2; 119.5; 127.3; 127.5; 130.7; 131.6; 160.2. m/z (LC/MS) = 189.

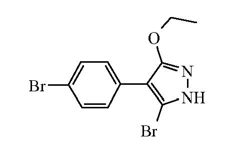

5-bromo-4-(4-bromophenyl)-3-ethoxy-1H-pyrazole (**42**): Compound **34** (0.36 g, 1.92 mmol) and bromine (0.3 mL, 5.77 mmol) were heated to reflux in ethanol (50 mL) for 5 minutes. This was concentrated to dryness, diluted in ethyl acetate washed with sodium sulfite, brine, dried over sodium sulfate and concentrated to dryness. The residu was purified by chromatography over neutral alumina containing 1.5 % of water (dichloromethane and then dichloromethane / ethanol 99/1) to yield compound **42** as a white solid (0.07 g, 49 %). ¹H (CDCl₃): 1.49 (t, 3H, J = 7.0); 4.40 (q, 2H, J = 7.0); 7.48 (m, 2H); 7.56 (m, 2H); 7.68 (s, 1H). ¹³C (CDC1₃): 14.9; 65.2; 106.2; 119.5; 127.3; 127.5; 130.7; 131.6; 160.2. HRMS: Calcd. for C₁₁H₁₀N₂O⁷⁹Br₂ + H : 344.9238. Found: m/z, 344.9243.

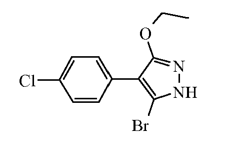

5-bromo-4-(4-chlorophenyl)-3-ethoxy-1H-pyrazole (**43**): 4-(4-chlorophenyl)-3-ethoxy-1H-pyrazole (**35**) (0.18 g, 0.81 mmol) and potassium carbonate (0.12 g, 0.89 mmol) were dissolved in ethanol (10 mL) and water (3 mL). To this was added bromine (0.046 mL, 0.89 mmol) and the solution was stirred at 25 °C overnight. This was diluted in water, extracted with ethyl acetate, dried over sodium sulfate and concentrated to dryness. The residu was then purified by a chromatography over silica gel (dichloromethane) the fraction containing **43** was then recrystallized in cyclohexane to yield pure **43** as white crystals (0.03 g, 13 %). ¹H (CDCl₃): 1.47 (t, 3H, J = 7.0); 4.32 (q, 2H, J = 7.0); 7.39 (m, 2H); 7.59 (s, 2H). ¹³C (CDCl₃): 14.7; 65.0; 105.9; 112.6; 120.1; 128.7; 129.6; 132.8; 160.0. HRMS: Calcd. for C₁₁H₁₃N₂O³⁵Cl⁷⁹Br + H : 302.9721. Found: m/z, 302.9715.

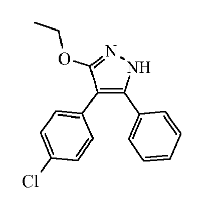

4-(4-chlorophenyl)-3-ethoxy-5-phenyl-1H-pyrazole (**44**): In a 5 mL biotage-adapted tube, 5-bromo-4-(4-chlorophenyl)-3-ethoxy-1H-pyrazole (0.03 g; 0.1 mmol), phenyl boronic acid (0.018 g; 0.15 mmol), cesium carbonate (0.081 g; 0.25 mmol) were dispersed in a mixture of propanol (1 mL) and water (1 mL). The oxygen was removed by a slow stream of argon and [1,1'-bis(diphenylphosphino)ferrocene] dichloropalladium complexed with dichloromethane (0.004g, 0.005 mmol) was then added before sealing the tube. This was heated in the microwave oven for 40 minutes at 120 °C. The mixture was concentrated to dryness. The residue was purified by a chromatography over silica gel (dichloromethane / ethanol 99-1) to yield compound **44** as a solid (0.010 g; 73 %). ¹H (CDCl₃): 1.42 (t, 3H, J = 7.0); 4.34 (q, 2H, J = 7.0); 7.28 (m, 2H); 7.31 (s, 2H); 7.39 (m, 5H) . ¹³C (CDCl₃): 14.9; 64.8; 103.6; 120.0; 127.8; 128.4; 129.0; 129.9; 130.1; 130.3; 131.9; 141.4; 161.3.

### Results

Unexpectedly, bromination of compound **34,** turned out to lead to the 4-brominated derivative **41.** Further work led to the bisbrominated compound **42**. However, little selectivity was observed in our 5-arylation attempts under Suzuki reaction conditions. On the other hand, bromination of the 4-chloroderivative **35** gave the 5-bromo-bearing compound **43.** This NH-bearing 5-bromopyrazole, upon coupling with phenyl-boronate led the expected 4.5-bisaryl derivative **44.**

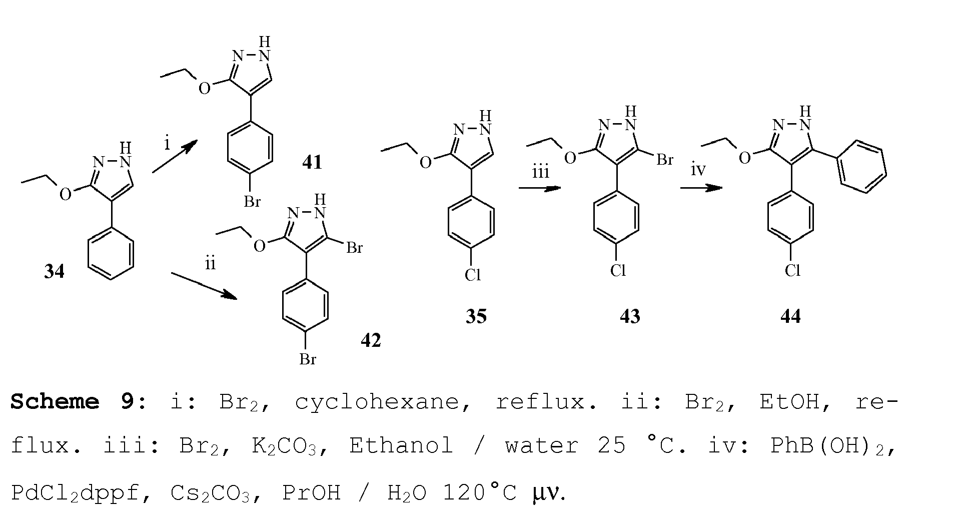

### Example 6: Acidic cleavage of the ethoxy group of 3-ethoxypyrazoles

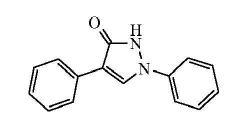

1,4-diphenyl-1H-pyrazol-3(2H)-one (**45**): In a 60 mL roundbottomed thick glass tube fitted with a PTFE-faced screwcap, a mixture of the considered ethoxypyrazole (1 mmol) was degassed with argon. Following this, 33 % hydrogen bromide in acetic acid (1.0 mL) was added; the tube was tightly closed and was heated at 140 °C for 2 hours. The resulting reaction mixture then precipitated in water to give compound **45** in an 83 % yield as a white powder. ¹H (DMSO-*d₆*): 7.17 (m, 2H); 7.43 (m, 2H); 7.39 (m, 2H); 7.47 (m, 2H); 7.77 (m, 4H); 8.76 (s, 1H); 10.93 (s(l), 1H). ¹³C (DMSO-*d₆*): 109.0; 117.2; 125.3; 125.9; 126.0; 126.1; 128.9; 129.9; 140.0; 160.4. HRMS: Calcd. for C₁₅H₁₂N₂O + H : 237.1028. Found: m/z, 237.1043.

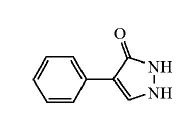

4-phenyl-1H-pyrazol-3(2H)-one (**46**): In a 60 mL roundbottomed thick glass tube fitted with a PTFE-faced screwcap, a mixture of the considered alkoxypyrazole (1 mmol) was degassed with argon. Following this, 33 % hydrogen bromide in acetic acid (1.0 mL) was added; the tube was tightly closed and was heated at 140 °C for 3 hours. The resulting solution was concentrated to dryness, made basic with methanolic ammonia, cocnntrated again and the residue was purified by a chromatography over silica gel (dichloromethane / ethanol from 99/1 to 94/6). ¹H (DMSO-*d₆*): 7.10 (m, 1H); 7.29 (m, 2H); 7.68 (m, 2H); 7.90 (s, 1H).

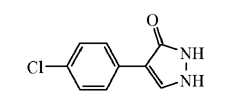

4- (4-chlorophenyl) -1H-pyrazol-3 (2H) -one (**47**) : By using the protocol described above, compound **47** was obtained as a white powder. ¹H (DMSO-*d₆*): 7.36 (d, 2H, J = 8.6); 7.70 (d, 2H, J = 8.6); 7.94 (s, 1H). ¹³C (DMSO-*d₆*): 104.5; 126.9; 128.1; 128.7; 129.3; 132.7; 158.8. HRMS: Calcd. for C₈H₇N₂O³⁵Cl + H : 195.0325. Found: m/z, 195.0264.

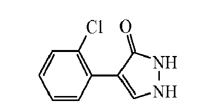

4-(2-chlorophenyl)-1H-pyrazol-3(2H)-one (**48**) : By using the protocol described above, compound **48** was obtained as a white powder. ¹H (DMSO-*d₆*): 7.19 (m, 1H); 7.33 (m, 1H); 7.45 (m, 1H); 7.65 (m, 1H); 7.80 (s, 1H). ¹³C (DMSO-*d₆*): 101.4; 126.8; 127.1; 129.6; 129.7; 130.8; 131.1; 131.4; 158.4.

### Results

The ethoxy groups protecting the oxygen moiety of these alkoxypyrazoles were cleaved by the use of hydrogenbromide in acetic acid. It was found that oxygen was detrimental to these reactions which led us to conduct these hydrolyses in a sealed reactor under an argon atmosphere. As an illustration of this process, compounds **45-48** were thus obtained from the corresponding 3-ethoxypyrazoles.

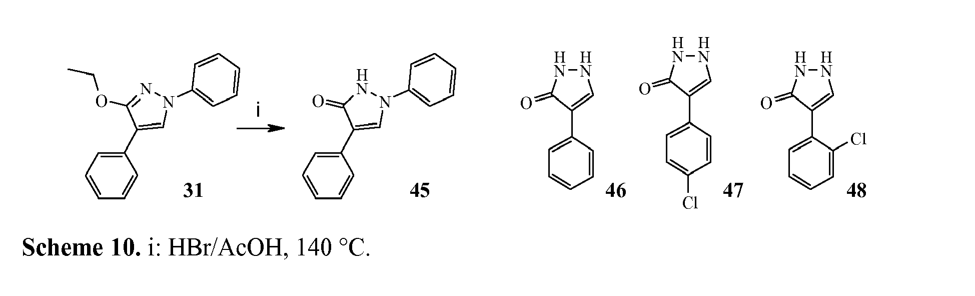

### REFERENCES

1. Miyaura, N.; Suzuki, A. Chem. Rev. 1995, 95, 2457-2483.
2. Miyaura, N. Top. Curr. Chem. 2002, 219, 12-59.
3. Kimata, A.; Nakagawa, H.; Ohyama, R.; Fukuuchi, T.; Ohta, S.; Doh-ura, K.; Suzuki, T.; Miyata, N. J. Med. Chem. 2007, 50, 5053-5056.
4. Kees, K.L.; Fitzgerald, J.J.; Steiner, K.E.; Mattes, J.F.; Mihan, B.; Tosi, T.; Mondoro, D.; McCaleb, M.L. J. Med. Chem. 1996, 39, 3920-3928.
5. Himly, M.; Jahn-Schmid, B.; Pittertschatscher, K.; Bohle, B.; Grubmayr, K.; Ferreira, F.; Ebner, H.; Ebner, C. J. Allergy Clin. Immunol. 2003, 111, 882-888.
6. Kees, K.L.; Caggiano, T.J.; Steiner, K.E.; Fitzgerald, J.J.; Kates, M.J.; Christos, T.E.; Kulishoff, J.M.; Moore, R.D.; McCaleb, M.L. J. Med. Chem. 1995, 38, 617-628.
7. Otterness, I.G. Clin. Exp. Immunol. 1981, 46, 332-339.
8. Ishimaru, T. Yakugaku Zasshi 1957, 77, 800-802. See Chem. Abstr. 1957: 17893.
9. Braibante, M.E.F.; Braibante, H.T.S.; de Carvalho Tavares, L.; Rohte, S.F.; Costa, C.C.; Morel, A.F.; Stüker, C.Z.; Burrow, R.A. Synthesis 2007, 2485-2490.
10. Li, L.; Chen, X.; Cutler, S.T. Patent WO 2001 082930 *see* Chem. Abstr. 2001 135: 344479
11. Willson, T.S.; Henke, B.R.; Momtahen, T.M.; Garrison, D.T.; Moore, L.B.; Geddie, N.G.; Baer, P.G. Bioorg. Med. Chem. Lett. 1996, 6, 1047-1050.
12. Cottineau, B.; Toto, P.; Marot, C.; Pipaud, A.; Chenault, J. Bioorg. Med. Chem. Lett. 2002, 12, 2105-2108.
13. Wasserman, H.H.; Han, W.T. Tetrahedron Lett. 1984, 25, 3743-3746.
14. McElvain, S. M.; Burkett, H. J. Am. Chem. Soc. 1942, 64, 1831-1836.
15. Chan, D.M.T.; Monaco, K.L.; Wang, R.-P.; Winters, M.P. Tetrahedron Lett. 1998, 39, 2933-2936.
16. Lam, P.Y.S.; Clark, C.G.; Saubern, S.; Adams, J.; Winters, M.P.; Chan, D.M.T.; Combs, A. Tetrahedron Lett. 1998, 39, 2941-2944.
17. Lam, P.Y.S.; Vincent, G.; Clark, C.G.; Deudon, S.; Jadhav, P.K. Tetrahedron Lett. 2001, 42, 3415-3418.
18. Chan, D.M.T.; Lam, P.Y.S. Recent advances in copper-promoted C-heteroatom bond cross-coupling reactions with boronic acids and derivatives. In Boronic acids; Wiley-VCH Ed., 2005; pp. 205-240.
19. Subasinghe, N.L.; Ali, F.; llig, C.R.; Rudolph, M.J.; Klein, S.; Khalil, E.; Soll, R.M.; Bone, R.F.; Spurlino, J.C.; DesJarlais, R.L.; Crysler, C.S.; Cummings, M.D.; Morris, P.E.; Kilpatrick, J.M.; Babu, Y.S. Bioorg. Med. Chem. Lett. 2004, 14, 3043-3047.
20. Dyckman, A.; Das, J.; Leftheris, K.; Liu, C.; Moquin, R.V.; Wrobleski, S.T. Patent WO 2004098518 *see* Chem. Abstr. 2004 141:424181
21. Ohta, T.; Fujisawa, H.; Nakai, Y.; Furukawa, I. Bull. Soc. Chim. Jpn. 2000, 73, 1861-1864.
22. Ley, S.V.; Thomas, A.W. Angew. Chem., Int. Ed. 2003, 42, 5400-5449.
23. Jurcak, J.G.; Barrague, M.; Gillespy, T.A.; Edwards, M.L.; Musick, K.Y.; Weintraub, P.M.; Du, Y.; Dharanipragada, R.M.; Parkar, A.A. Patent WO 2005 026175 *see* Chem. Abstr. 2005 142: 316835
24. Guillou, S.; Janin, Y.L. J. Heterocyclic. Chem. 2008**,** in print.
25. Chu, L.; Fisher, M.H.; Goulet, M.T.; Wyvratt, M.J. Tetrahedron Lett. 1997, 38, 3871-3874.
26. Drysdale, M.J.; Dymock, B.W.; Barril-Alonso, X.; Workman, P.; Pearl, L.H.; Prodromou, C.; MacDonald, E. Patent WO 03 55,860 *see* Chem. Abstr. 2003 139: 101122k
27. Beaulieu, P.L.; Gillard, J; Bykowski, D.; Brochu, C.; Dansereau, N.; Duceppe, J.S.; Haché, B.; Jakalian, A.; Lagacé, L.; LaPlante, S.; McKercher, G.; Moreau, E.; Perreault, S.; Stammers, T.; Thauvette, L.; Warrington, J.; Kukolj, G. Bioorg. Med. Chem. Lett. 2006, 16, 4987-4993.
28. Bernal, P.; Tamariz, J. Helv. Chim. Acta 2007, 90, 1449-1454.
29. Kwong, F.Y; Klapars, A.; Buchwald, S.L. Org. Lett. 2002, 4, 581-584.
30. Conesa, L.I.; Cawley, M.J.; Cloke, F.G.N.; Arentsen, K.; Scott, J.S.; Pearson, S.E.; Hayler, J.; Caddick, S. J. Organomet. Chem. 2005, 690, 5841-5848.
31. Knorr, L. Ber. Dtsch. Chem. Ges. 1895, 28, 706-714.
32. Stanovik, E.; Svete, J. Product class 1: pyrazoles. In Science of Synthesis; Neier, R. Ed.; Thieme, 2002; Vol. 12; pp. 15-225.
33. Varvounis, G.; Fiamegos, Y.; Pilidis, G. Pyrazol-3-ones, Part 1: synthesis and applications. In Adv. Heterocyclic Chem.; Katritzky, A. R. Ed.; Academic Press, 2001; Vol. 80; pp. 74-144.
34. Bau, B.; Hofmann, T.; Kloss, J.; Neunhoeffer, H. Sci. Pharm. 1998, 66, 119-136.
35. Sustmann, R.; Sicking, W.; Felderhoff, M.; Tabarsi, P. Tetrahedron 1990, 46, 783-792.

## Claims

1. A process for the preparation of alkoxypyrazoles of formula (I) or (I') 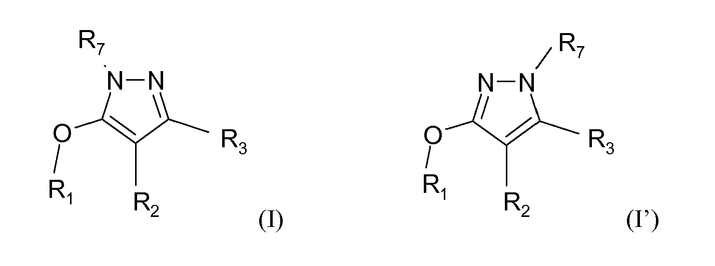 in which
R₁ stands for a radical selected from the group consisting of C₁-C₆ alkyl, C₂-C₆ alkenyl, C₂-C₆ alkynyl and (C₆-C₁₀) aryl (C₁-C₆) alkyl;
R₂ stands for a radical selected from the group consisting of hydrogen, halogen, -C(O)-O-(C₁-C₆)alkyl, - C(O)-O-(C₂-C₆)alkenyl, -C(O)-O-(C₂-C₆)alkynyl;
R₃ stands for a radical selected from the group consisting of hydrogen, halogen, C₁-C₆ alkyl, C₂-C₆ alkenyl, C₂-C₆ alkynyl and (C₆-C₁₀)aryl(C₁-C₆)alkyl, each optionally substituted by R₅;
R₅ is a radical selected from the group consisting of halogen, oxygen, hydroxyl, C₁-C₆ alkyl and C₁-C₆ alkoxy;
R₇ stands for a radical selected from the group consisting of H, C₁-C₆ alkyl, C₆-C₁₀ aryl, (C₆-C₁₀)aryl(C₁-C₆)alkyl, C₆-C₁₀ heteroaryl or (C₆-C₁₀)heteroaryl(C₁-C₆)alkyl, each optionally substituted by a halogen;
comprising the successive following steps:
(a) reacting a derivative of formula (II) 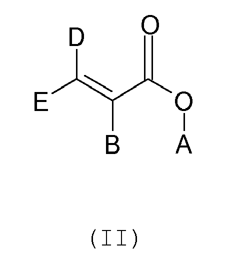
in which A stands for a radical selected from the group consisting of C₁-C₆ alkyl, C₂-C₆ alkenyl, C₂-C₆ alkynyl and (C₆-C₁₀)aryl(C₁-C₆)alkyl;
B stands for a radical selected from the group consisting of hydrogen, -C(O)-O-(C₁-C₆)alkyl, -C(O)-O-(C₂-C₆)alkenyl, -C(O)-O-(C₂-C₆)alkynyl;
D stands for a radical selected from the group consisting of hydrogen, C₁-C₆ alkyl, C₂-C₆ alkenyl, C₂-C₆ alkynyl and (C₆-C₁₀)aryl(C₁-C₆)alkyl;
E stands for a radical O-F or NR_{α}R_{β},
F stand for a radical selected from the group consisting of H, C₁-C₆ alkyl, C₂-C₆ alkenyl, C₂-C₆ alkynyl and (C₆-C₁₀)aryl(C₁-C₆)alkyl;
R_{α} and R_{β}, identical or different, stand for a radical selected from the group consisting of H, C₁-C₆ alkyl or C₆-C₁₀ aryl or R_{α} and R_{β} form together an C₄-C₆ alkylene radical, an C₄-C₆ alkenylene, an C₄-C₆ heteroalkylene radical or an C₄-C₆ heteroalkenylene radical;
with 1 to 1.5 molar equivalents of R₇-NH-NH₂, xHX₂, in which R₇ as defined for compound of formula (I) or (I'), X₂ is Cl or Br and x is 1 or 2;
in an alkylalcohol R₈0H as a solvent, in which R₈ is a radical selected from the group consisting of C₁-C₆ alkyl, C₂-C₆ alkenyl, C₂-C₆ alkynyl and (C₆-C₁₀)aryl(C₁-C₆)alkyl, (C₆-C₁₀)heteroaryl(C₁-C₆)alkyl;
to obtain compounds of formula (III) or (III') 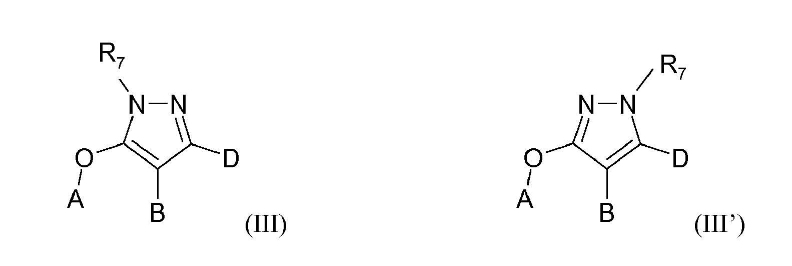
(b) optionally reacting said alkoxypyrazole of formula (III') wherein R₇ = H obtained in step (a) with X to prepare compounds of formula (IV) or (IV') 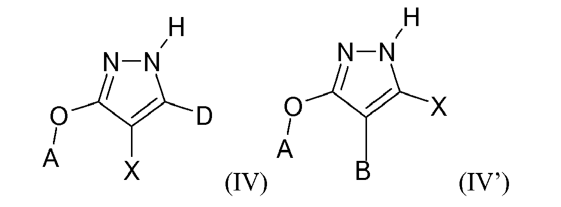 in which
X represents Br, Cl, I or F;
A, B and D are as defined above;
(c) recovering the alkoxypyrazole of formula (I) or (I').

2. The process according to claim 1, wherein in step (a) the compound of formula (II) is reacted with 1.05 molar equivalents of R₇-NH-NH2, xHX₂, in which R₇, X₂ and x are as defined in claim 1.

3. The process according to claims 1 or 2, wherein in step (a) the reaction is run in boiling ethanol.

4. The process according to any one of the preceding claims, wherein E is NR_{α}R_{β}, X₂ is Cl and x is 2.

5. The process according to any one of claims 1 to 3, wherein E is O-F, F is C₁-C₆ alkyl, X₂ is Cl and x is 1.

6. The process according to any one of the preceding claims , wherein R₇ is a hydrogen or a methyl, benzyl or phenyl group.

7. The process according to any one of the preceding claims, wherein the reaction in step (a) is run at the boiling point of the solvent.

8. The process according to claim 5, wherein the reaction in step (a) is run for 16 hours.

9. The process according to any one of the preceding claims, wherein R₈ is an ethyl group.

10. The process according to any one of the preceding claims, wherein A stands for a radical selected from the group consisting of C₁-C₆ alkyl.

11. The process according to any one of the preceding claims, wherein B stands for a radical selected from the group consisting of hydrogen, -COOH or -COOEt.

12. The process according to any one of the preceding claims, wherein D stands for a radical selected from the group consisting of hydrogen or methyl.

13. The process according to any one of the preceding claims, wherein E stands for a radical selected from the group consisting of NMeMe or O-ethyl.

14. Alkoxypyrazoles of formula I or I' obtainable by the process according any one of the preceding claims, except compounds of formula (I') wherein simultaneously
R₁ is ethyl, R₂ is H, R₃ is phenyl, R₇ is H;
R₁ is ethyl, R₂ is -COOH,-COOEt or H, R₃ and R₇ are H;
R₁ is methyl, R₂, R₃ and R₇ are H;
R₁ is ethyl, R₂ is-COOEt, R₃ is H, R₇ is methyl
and compounds of formula (I) wherein simultaneously
R₁ is ethyl, R₂ is -COOEt, R₃ is H and R₇ is phenyl;
R₁ is ethyl, R₂ is -COOEt, R₃ is H and R₇ is methyl.

15. Alkoxyppyrazoles of formula (V) or (V') 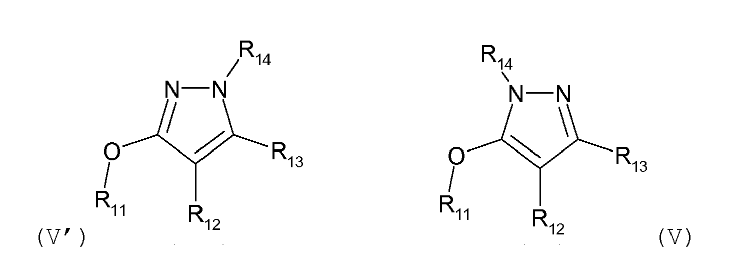 in which
R₁₁ stands for a radical selected from the group consisting of methyl or ethyl,
R₁₂ stands for a radical selected from the group consisting of a hydrogen, halogen, benzyl, a phenyl optionally substituted by Cl or Br, -COOH,-COOEt;
R₁₃ stands for a radical selected from the group consisting of hydrogen, halogen, methyl, phenyl, benzyl, morpholino,
R₁₄ stands for a radical selected from the group consisting of hydrogen, phenyl, benzyl, mesyl, -CH₃SO₂ or methyl,
except compounds of formula (V') wherein simultaneously
R₁₁ is ethyl, R₁₂ is H, R₁₃ is phenyl, R₁₄ is H;
R₁₁ is ethyl, R₁₂ is -COOH,-COOEt or H, R₁₃ and R₁₄ are H;
R₁₁ is methyl, R₁₂, R₁₃ and R₁₄ are H;
R₁₁ is ethyl, R₁₂ is-COOEt, R₁₃ is H, R₁₄ is methyl
and compounds of formula (V) wherein simultaneously
R₁₁ is ethyl, R₁₂ is -COOEt, R₁₃ is H and R₁₄ is phenyl;
R₁₁ is ethyl, R₁₂ is -COOEt, R₁₃ is H and R₁₄ is methyl.

16. Alkoxypyrazoles of formula (V) or (V') according to claim 14,in which
R₁₁ stands for a radical selected from the group consisting of methyl or ethyl,
R₁₂ stands for a radical selected from the group consisting of a hydrogen, halogen, -COOH,-COOEt;
R₁₃ stands for a radical selected from the group consisting of hydrogen, halogen, methyl,
R₁₄ stands for a radical selected from the group consisting of hydrogen, phenyl, benzyl or methyl,
except compounds of formula (V') wherein simultaneously
R₁₁ is ethyl, R₁₂ is H, R₁₃ is phenyl, R₁₄ is H;
R₁₁ is ethyl, R₁₂ is -COOH,-COOEt or H, R₁₃ and R₁₄ are H;
R₁₁ is methyl, R₁₂, R₁₃ and R₁₄ are H;
R₁₁ is ethyl, R₁₂ is-COOEt, R₁₃ is H, R₁₄ is methyl
and compounds of formula (V) wherein simultaneously
R₁₁ is ethyl, R₁₂ is -COOEt, R₁₃ is H and R₁₄ is phenyl;
R₁₁ is ethyl, R₁₂ is -COOEt, R₁₃ is H and R₁₄ is methyl.

17. Alkoxypyrazoles of formula (V'),in which
R₁₁ is ethyl, R₁₃ is phenyl, R₁₄ is H, R₁₂ is -COOH,-COOEt, 4-BrC₆H₄ or 4-ClC₆H₄;
R₁₁ is ethyl, R₁₃ and R₁₄ are H, R₁₂ is phenyl, I, 4-ClC₆H₄, 2-ClC₆H₄, -CH₂CH₂C₆H₅, 4-BrC₆H₄ or -CH₂C₆H₅;
R₁₁ is ethyl, R₁₂ is COOEt, R₁₄ is H, R₁₃ is Br, I, methyl;
R₁₁ is ethyl, R₁₂ is -COOEt, R₁₄ is phenyl and R₁₃ is H, I, Br or phenyl;
R₁₁ is ethyl, R₁₂ is I, R₁₃ is H, R₁₄ is phenyl;
R₁₁ is ethyl; R₁₃ is H, R₁₄ is phenyl, R₁₂ is H or phenyl;
R₁₁ is ethyl, R₁₃ is Br, R₁₄ is H, R₁₂ is 4-BrC₆H₄ or 4 -ClC₆H₄;
R₁₁ is ethyl, R₁₂ is -COOH, R₁₃ and R₁₄ are phenyl;
R₁₁ is ethyl, R₁₂ is -COOEt, R₁₃ is H, R₁₄ is -CH₂C₆H₅;
R₁₁ is ethyl, R₁₂ is I, R₁₃ is H, R₁₄ is -CH₃SO₂;
R₁₁ is ethyl, R₁₂ is -COOEt, R₁₃ is morpholino, R₁₄ is H;
R₁₁ is ethyl, R₁₂ is -COOEt, R₁₃ is I, R₁₄ is -CH₃SO₂;
R₁₁ is ethyl, R₁₂ is H, R₁₃ is I, R₁₄ is H;
R₁₁ is ethyl, R₁₂ is H, R₁₃ is benzyl, R₁₄ is H;
R₁₁ is ethyl, R₁₂ is -COOH, R₁₃ is H, R₁₄ is phenyl;
R₁₁ is ethyl, R₁₂ is H, R₁₃ is phenyl, R₁₄ is phenyl;
R₁₁ is ethyl, R₁₂ is I, R₁₃ is H, R₁₄ is mesyl;
R₁₁ is ethyl, R₁₂ is -COOEt, R₁₃ is I, R₁₄ is mesyl.

18. Alkoxypyrazoles of formula (V),in which
R₁₁ is ethyl; R₁₃ is H, R₁₄ is phenyl, R₁₂ is H or I;
R₁₁ is ethyl; R₁₂ is -COOEt, R₁₃ is I or Br, R₁₄ is phenyl;
R₁₁ is ethyl; R₁₂ is -COOEt, R₁₃ is H, R₁₄ is -CH₂C₆H₅;
R₁₁ is ethyl; R₁₂ is I, R₁₃ is H, R₁₄ is mesyl;
R₁₁ is ethyl; R₁₂ is -COOEt, R₁₃ is I, R₁₄ is mesyl;
R₁₁ is ethyl, R₁₂ is -COOEt, R₁₃ is I, R₁₄ is -CH₃SO₂;
R₁₁ is ethyl, R₁₂ is I, R₁₃ is H, R₁₄ is -CH₃SO₂.
